(19) Europäisches Patentamt
European Patent Office
Office européen des brevets

(11) Publication number: **0 256 424 B1**

(12) # EUROPEAN PATENT SPECIFICATION

(45) Date of publication of patent specification: **25.09.91**

(51) Int. Cl.⁵: **C12N 15/00**, C12N 1/20, A61K 37/66

(21) Application number: **87111281.9**

(22) Date of filing: **04.08.87**

(54) **Homogeneous recombinant immune interferon fragments and pharmaceutical compositions containing same.**

(30) Priority: **13.08.86 GB 8619725**

(43) Date of publication of application:
**24.02.88 Bulletin 88/08**

(45) Publication of the grant of the patent:
**25.09.91 Bulletin 91/39**

(84) Designated Contracting States:
**AT BE CH DE ES FR GB GR IT LI LU NL SE**

(56) References cited:
**EP-A- 0 145 174**
**EP-A- 0 146 354**
**EP-A- 0 147 175**
**EP-A- 0 161 504**
**EP-A- 0 166 993**

**T. Arakawa et al. J. Biol. Chem. 261 (8), 8534-8539 (1986)**

**T. Arakawa et al, B.B.R.C., 136(2), 679-684 (1986)**

(73) Proprietor: **F. HOFFMANN-LA ROCHE AG**
**Postfach 3255**
**CH-4002 Basel(CH)**

(72) Inventor: **Döbeli, Heinz, Dr.**
**Lupsingerstrasse 22**
**CH-4417 Ziefen(CH)**
Inventor: **Gentz, Reiner, Dr.**
**Am Hochgericht 34**
**W-7888 Rheinfelden(DE)**
Inventor: **Hochuli, Erich, Dr.**
**Kirchackerstrasse 25**
**CH-4411 Arisdorf(CH)**

(74) Representative: **Lederer, Franz, Dr. et al**
**Patentanwalt Dr. Franz Lederer Lucile-Grahn-Strasse 22**
**W-8000 München 80(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to homogeneous recombinant human immune interferon fragments having 6 to 11 amino acids deleted at the carboxy terminus as compared to full-length mature recombinant human immune interferon (amino acid sequence shown in Figure 1). The invention also relates to replicable microbial expression vehicles comprising a nucleotide sequence encoding a recombinant immune interferon fragment defined above, to microorganisms transformed with these expression vehicles and to processes for the preparation of said interferon fragments and said microorganisms. The invention further relates to pharmaceutical compositions containing one or more of these recombinant immune interferon fragments and to the use of these recombinant immune interferon fragments and pharmaceutical compositions for the treatment of various disease states.

Natural human immune interferon (IFN-γ) is produced upon mitogenic stimulation of lymphocytes. It exhibits antiviral and anti-proliferative activity. This activity is pH 2 labile. The functional form of the immune interferon may be a multimer, most likely a dimer, a trimer or a tetramer (Pestka et al., J. Biol. Chem. 258, 9706-9709 [1983]).

It has been found that immune interferon is encoded in the human genome by a single gene which codes for a precursor-polypeptide of 166 amino acid residues (Derynck et al., Nucleic Acids Res. 10, 3605-3615 [1982]). Post-translational processing was believed to result in a polypeptide comprising 146 amino acids and having a Cys-Tyr-Cys-Gln-... N-terminal sequence.

Using methods of the recombinant DNA technology (see for example Maniatis et al., "Molecular cloning - A laboratory manual", Cold Spring Harbor Laboratory, 1982) expression vectors coding for a recombinant immune interferon comprising these 146 amino acids have been constructed. Upon introduction of these expression vectors into a microbial host, recombinant immune interferon polypeptides consisting of 147 amino acids, namely the 146 amino acids mentioned above and an additional N-terminal methionine, are synthesized. The additional methionine derives from the mRNA translational start signal AUG which codes for the amino acid methionine. In the parental human gene this translational start signal is situated in front of the signal peptide. This signal peptide is cleaved off upon post-translational processing, together with the methionine derived from the translational start signal leading to methionine-free mature immune interferon consisting of 146 amino acids. Prokaryotic hosts are not able to cleave the signal peptide in front of the human immune interferon precursor polypeptide at the right position. Therefore the coding sequence for the signal peptide in the immune interferon gene has to be removed by genetic engineering in order to express the immune interferon in mature form. The ATG codon directly in front of the coding sequence for the mature polypeptide results in the N-terminal methionine present in recombinant human immune interferon. In spite of the additional N-terminal methionine and despite the fact that it is not glycosylated the recombinant immune interferon exhibits antiviral and anti-proliferative activity coupled with pH 2 lability comparable to the immune interferon isolated from human lymphocytes (Gray et al., Nature 295, 503-508 [1982]). Later on Rinderknecht et al., (J. Biol. Chem. 259, 6790-6797 [1984]) have shown that immune interferon purified from induced human peripheral blood lymphocytes consists of a polypeptide having 143 amino acid residues, lacking the Cys-Tyr-Cys- sequence and having a pyroglutamate residue at its N-terminus. Some heterogeneity at the carboxy terminus was observed. Reengineering of the original recombinant immune interferon gene led to expression vectors coding for mature recombinant immune interferon comprising these 143 amino acids and having as outlined above an additional methionine at the N-terminus. Comparison of the biological activities of the recombinant human immune interferons with or without the Cys-Tyr-Cys- sequence showed, that the deletion of the Cys-Tyr-Cys- sequence led to a twofold higher antiviral activity. (European Patent Application No. 146 354, published on 26.6.85). Moreover it is shown in European Patent Applica- tion No. 146 354 that immune interferon fragments generated by limited tryptic digestion of mature reccmbinant human immune interferon, had decreased antiviral activity, compared to a recombinant immune interferon preparation consisting of a mixture of interferon polypeptides (= reference material) having 139 or 143 amino acids (ratio 98:2). For example a fragment consisting of 131 amino acids, lacking 12 C-terminal amino acids, showed 40-50% specific activity compared to the above mentioned reference material. Fragments with additional 3 or 6 amino acids removed from the C-terminus had 6-9 or 1% respectively of the specific activity of the reference material. Therefore in view of the reduced activity exhibited by the immune interferon frag- ments exemplified in the above mentioned European Patent Application, it can be concluded that all fragments lacking terminal amino acids will have a lower antiviral activity than the above-mentioned reference material or the mature recombinant human immune interferon with 143 amino acids lacking the Cys-Tyr-Cys- sequence at the N-terminus. On the other hand these immune interferon fragments have been generated only by limited tryptic digestion. Trypsin catalyzes the hydrolysis of peptide bonds, whose carbonyl function is donated by a basic amino acid, usually arginine or lysine. There are 20 lysine residues and 8 arginine residues in the amino acid sequence of immune interferon. The amount of the individual fragments generated by trypsin depends on the accessibility of the peptide bond which has to be cut to generate the fragment. Nevertheless, limited proteolytic digestion leads to a wide spectrum of fragments and the purification of a specific fragment to homogeneity from such a mixture is highly problematic in particular since polypeptides having essentially the same amino acid sequence but differing in size by a few amino acids would have to be separated. Under the prevailing conditions the fractionation by HPLC described in European Patent Application No. 146 354 is not considered as adequate for purifying the immune interferon fragments exemplified therein to homogeneity, that means that they are obtained substantially free from other immune interferon fragments differing in size. Moreover, due to the specificity of trypsin, the immune interferon fragments of the present invention having non-basic C-terminal amino acids like serine, glutamine, methionine, leucine or phenylalanine cannot be generated by tryptic digestion. This may be the reason why only immune interferon fragments with 125, 129, 131 and 139 amino acids, having either lysine or arginine as C-terminal amino acid are exemplified in European Patent Application No. 146 354. Although a method for the preparation of immune interferon fragments by recombinant DNA technology is generally described in the above mentioned European Patent Application, recombinant human immune interferon fragments comprising 132 to 137 amino acids counted from the first glutamine residue in the amino acid sequence shown in Figure 1, have been prepared for the first time in homogeneous form by the methods of the present invention. Most surprisingly it has been found that these homogeneous recombinant immune fragments have an increased specific antiviral activity compared to full length mature recombinant immune interferon. Similarly it was observed that the other biological activities exhibited by immune interferon (reviewed by Trinchieri et al., Immunology Today 6, 131-136 [1985]) are also increased for the recombinant immune interferon fragments of the present invention in comparison to the mature recombinant immune interferon. Pharmaceutical compositions containing one or more of the recombinant immune interferon fragments of the present invention can be used for the treatment of various disease states. Examples of such disease states are: viral infections, neoplastic diseases or rheumatoid arthritis.

The present invention therefore provides homogeneous recombinant immune interferon fragments exhibiting a specific antiviral activity higher than mature recombinant immune interferon and having the amino acid sequence

X-Y-Asp-Pro-Tyr-Val-Lys-Glu-Ala-Glu-Asn-Leu-Lys-Lys-Tyr-Phe-
Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-Lys-Asn-Phe-
Lys-Asp-Asp-Gln-Ser-Ile-Gln-Lys-Ser-Val-Glu-Thr-Ile-Lys-Glu-
Asp-Met-Asn-Val-Lys-Phe-Phe-Asn-Ser-Asn-Lys-Lys-Lys-Arg-Asp-
Asp-Phe-Glu-Lys-Leu-Thr-Asn-Tyr-Ser-Val-Thr-Asp-Leu-Asn-Val-
Gln-Arg-Lys-Ala-Ile-His-Glu-Leu-Ile-Gln-Val-Met-Ala-Glu-Leu-
Ser-Pro-Ala-Ala-Lys-Thr-Gly-Lys-Arg-Lys-Arg-Z

wherein either x is a methionine and Y is a glutamine residue or X is hydrogen and Y is a glutamine or a pyroglutamate residue, and Z is

Ser,
Ser-Gln,
Ser-Gln-Met,
Ser-Gln-Met-Leu,
Ser-Gln-Met-Leu-Phe or
Ser-Gln-Met-Leu-Phe-Arg.

These homogeneous recombinant immune interferon fragments have a significant higher biological activity compared to mature recombinant human immune interferon, that means to a full-length recombinant immune interferon consisting of 143 amino acids, having either a glutamine or a pyroglutamate residue at position 1 of its amino acid sequence. When the amino acid residue at position 1 is glutamine, the full-length recombinant immune interferon may contain optionally an additional methionine residue as 144th amino acid at its N-terminus. The additional methionine residue derives from the mRNA translational start signal AUG which codes for the amino acid methionine as outlined above. In expression systems like E.coli this methionine is not always processed away. The additional N-terminal methionine has been found not to impair the biological activity of most recombinant polypeptides (Winnacker, in "Gene und Klone", p. 255, VCH, Weinheim, Germany [1985]). Upon removal of the N-terminal methionine in recombinant immune interferon or its fragments the newly generated N-terminal glutamine residue may cyclize to the pyroglutamate form, again without any believed impairment of the biological activity. The recombinant immune interferon fragments of the present invention may be in multimerized form, preferably they are present as dimers, trimers or tetramers.

The recombinant immune interferon fragments of the present invention can be used to prepare pharmaceutical compositions consisting of a significantly reduced amount of said recombinant immune interferon fragments as compared to the amount of mature recombinant human immune interferon needed, to obtain essentially the same antiviral activity and a physiological compatible carrier. Such pharmaceutical compositions are useful for the treatment of various disease states. It is understood that amino acid substitutions, particularly single amino acid substitutions in the recombinant immune interferon fragments disclosed herein, giving rise to variants of the recombinant immune interferon fragments having higher specific activity compared to mature recombinant human immune interferon are possible. It is within the state of the art to introduce such substitutions in a recombinant polypeptide. More generally the present invention includes all recombinant immune interferon fragments having 6 to 11 amino acids deleted at the C-terminus as compared to a full-length mature recombinant human immune interferon or modifications and allelic variations thereof, which fragments exhibit biological activity greater than the full-length mature recombinant human immune interferon. The invention also provides replicable microbial expression vehicles comprising a nucleotide sequence encoding a recombinant immune interferon fragment operably linked to an expression control sequence and microorganisms transformed with such an expression vehicle. Moreover it provides pharmaceutical compositions containing one or more recombinant immune interferon fragments and a physiological compatible carrier. The invention further relates to processes for the preparation of recombinant immune interferon fragments through recombinant DNA technology, to processes for the preparation of a microorganism transformed with a replicable microbial expression vehicle mentioned above and to methods for the preparation of pharmaceutical compositions containing such recombinant immune interferon fragments. Furthermore the invention relates to the use of these interferon fragments and corresponding pharmaceutical compositions for the treatment of various disease states.

The present invention may be more readily understood by reference to the following detailed description of the invention when considered in connection with the accompanying drawings, wherein the following abbreviations and symbols are used:

B, E, H, S, Sa, Sc, X, Xb which indicate sites for restriction endonucleases BamHI, EcoRI, HindIII, SphI, SalI, ScaI, XhoI and XbaI, respectively.

In figures 2, 4 and 6 to 9

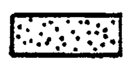

respresents promotors of the genes bla, lacI and neo;

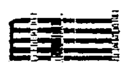

represents ribosomal binding sites of the genes bla, cat, neo and lacI;

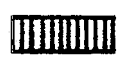

represents terminators $t_o$ and TI;

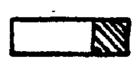

represents the regulatable promoter/operator element $P_{N25}X/O$;

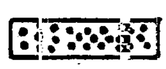

represents ribosomal binding site RBSII,SphI; → represents coding regions under control of this ribosomal binding site; ⇆ represents regions required for DNA replication (repl.);

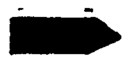

represents coding regions for dihydrofolate reductase (dhfr), chloramphenicol acetyltransferase (cat), β-lactamase (bla), lac repressor (lacI), neomycin phosphotransferase (neo), and interferon-γ (IFN-γ).

Figure 1

Amino acid sequence of mature recombinant human immune interferon (rIFN-γ) lacking the Cys-Tyr-Cys- sequence at the N-terminus and the nucleotide sequence encoding it. The HinfI site used for the construction of the recombinant immune interferon fragments is underlined. The N-terminal methionine derived from the prokaryotic translational start signal is put in parenthesis.

Figure 2

Schematic drawing of the plasmid pDS8/RBSII,SphI.

Figure 3

Nucleotide sequence of the XhoI/XbaI fragment of the plasmid pDS8/RBSII,SphI containing the regulatable promoter/operator element $P_{N25}x/o$, the ribosomal binding site RBSII,SphI, the dhfr-gene, terminator $t_o$, the cat-gene and terminator TI. The restriction endonuclease sites indicated in Fig. 2 are overlined and the region under control of RBSII,SphI encoding a dihydrofolate reductase polypeptide is underlined. In addition the pBR322 entity of pDS8/RBSII,SphI is schematically shown, where the given numbers refer to the nucleotide sequence of pBR322 (J.G. Sutcliffe, Cold Spring Harbor, Symp. Quant. Biol. 43, pp. 77-90 [1979]).

Figure 4

Schematic drawing of the plasmid pDMI,1.

Figure 5

DNA sequence of the plasmid pDMI,1. The restriction endonuclease sites indicated in Fig. 4 are overlined and the coding regions for neomycin phosphotransferase (neo) and lac repressor (lacI) are underlined.

Figure 6

Schematic description of the construction and isolation of fragment 1 containing the gene for mature recombinant human immune interferon (rIFN-γ) starting from plasmid pRC23/IFI-900 containing the gene coding for a recombinant human interferon consisting of 146 amino acids, having Cys-Tyr-Cys- as N-terminal sequence.

Figure 7

Integration of fragment 1 into pDS8/RBSII,SphI, resulting in pGLS. (Sc) in the schematic drawing of pGLS indicates the position were fragment 1 is linked to the ScaI cleavage site of pDS8/RBSII,SphI.

Figure 8

Schematic description of the construction of the EcoRI-HindIII-fragments F(-6), F(-8) and F(-11) coding for the C-terminus of three different recombinant immune interferon fragments using a HinfI-fragment of pGLS and the HinfI-HindIII-adapters A(-6), A(-8) and A(-11). The EcoRI-HindIII-fragments F(-7), F(-9) and F(-10) were constructed in similar way using the HinfI-HindIII-adapters

| | |
|---|---|
| A(-7) | AGTCAGATGCTGTTT<u>TAA</u><br>GTCTACGACAAAATTTCGA |
| A(-9) | AGTCAGATG<u>TAA</u><br>GTCTACATTTCGA |
| A(-10) | AGTCAG<u>TAA</u><br>GTCATTTCGA |

In the sequence given the relevant codons for translational termination are underlined.

Figure 9

Schematic description of the integration of fragments F(-6), F(-8) and F(-11) into the EcoRI- and HindIII-cleavage sites of pDS8/RBSII,SphI resulting in plasmids pIFN-γ(-6), pIFN-γ(-8) and pIFN-γ(-11). The fragments F(-7), F(-9) and F(-10) were integrated in similar way resulting in plasmids pIFN-γ(-7), pIFN-γ(-9) and pIFN-γ(-10).

Figure 10

Amino acid sequences of the gene products rIFN-γ, IFN-γ(-6), IFN-γ(-7), IFN-γ(-8), IFN-γ(-9), IFN-γ(-10) and IFN-γ(-11) which are obtained upon expression of pGLS, pIFN-γ(-6), pIFN-γ(-7), pIFN-γ(-8), pIFN-γ(-9), pIFN-γ(-10) and pIFN-γ(-11), respectively. (130aa) means the 130 amino acids from position 1 to position 130 (see Fig. 1)

Figure 11

Analysis of the mature human immune interferon and the human interferon fragments by SDS-polyacrylamide gelelectrophoresis. In lanes a, b, c and d (Fig. 11a, part A) and lanes 1 to 9 (Fig. 11b) lysates of E. coli M15 cells, harbouring pDMI,1 and in addition the expression vector indicated below, are shown:

| expression vector | Figure 11a | Figure 11 b |
|---|---|---|
| | lane | lane |
| pGLS | a | 1,9 |
| pIFN-γ(-4) | - | 2 |
| pIFN-γ(-5) | - | 3 |
| pIFN-γ(-6) | b | 4 |
| pIFN-γ(-8) | c | 5 |
| pIFN-γ(-9) | - | 6 |
| pIFN-γ(-10) | - | 7 |
| pIFN-γ(-11) | d | 8 |

In lanes a, b, c and d (Fig. 11a, part B) and lanes 10 to 17 and lane 20 (Fig. 11b) the purified interferon polypeptides isolated from the lysates a, b, c and d (Fig. 11a, part A) and lanes 1 to 9 (Fig. 11b) respectively are shown. The human immune interferon fragments IFN-γ(-14) and IFN-γ(-18) prepared by limited proteolysis are shown in lanes 18 and 19 respectively.

MW (Fig. 11a) and M (Fig. 11b) denote a protein size marker (BIO-RAD laboratories) consisting of proteins of 14.4 kd, 21.5 kd, 31 kd, 45 kd, 66.2 kd and 92.5 kd molecular weight (1 kd = 1'000 dalton.

Figure 12

Antiviral activity in relation to the carboxyterminal sequence of IFN-γ. The carboxyterminal sequence is given in the one letter code described by Dayhoff et al., ("Atlas of Protein Sequence and Structure", M.O. Dayhoff, ed., Vol. 5, p. 17, Natl. Biomed. Res. Found., Silver Spring, Maryland, U.S.A., [1979]) on the abscissa. The antiviral activity exhibited by the recombinant immune interferon fragments identified on the abscissa by the number of amino acids deleted at the carboxyterminus in comparison to the full-length mature recombinant human immune interferon (identified by "O") are plotted on the ordinate. Each point represents the geometric mean titer of at least six independent determinations. Bars indicate the corresponding 95 % confidence limits.

Figure 13

Comparison of the antiviral activity, the macrophage activation and the receptor binding capacity in relation to the carboxyterminal sequence of IFN-γ (for details see legend to Fig. 12 and text). The values for the antiviral activity, the macrophage activation and the receptor binding capacity of the immune interferon fragments in relationship to the corresponding values of mature recombinant immune interferon, which were arbitrarily taken as 1, are shown on a logarithmic scale. The absolute value for the macrophage activation of mature recombinant immune interferon was $5 \times 10^5$ U/mg.

In a preferred embodiment of the present invention recombinant immune interferon fragments can be prepared by selection of a suitable plasmid containing a full-length copy of the human immune interferon gene or allelic variants thereof. Translational stop codons can then be introduced in the region of the gene coding for the C-terminus of immune interferon either by site directed mutagenesis as described by Smith et al. (in "Genetic Engineering" 3, 1-32 [1981], J.K. Setlow, A. Hollaender eds., Plenum Press, New York) or by removing a suitable restriction fragment from this region and by replacing this restriction fragment with a synthetic DNA fragment coding for the desired C-terminus of the recombinant immune interferon fragment. In the latter method preferably two different restriction endonucleases are used to cut the starting plasmid, generating two different sticky ends. The synthetic DNA containing complementary sticky ends can then be integrated directly in the right orientation. In a next step the DNA sequence coding for the recombinant immune interferon fragment can be inserted in a replicable microbial expression vehicle, comprising an origin of replication, a promoter or promoter-operator and a sequence encoding a ribosomal binding site (RBS). Suitable replicable expression vehicles can be found in Maniatis et al. (supra) or in the examples of the present invention. Preferred replicable expression vehicles comprising a nucleotide sequence encoding a recombinant immune interferon fragment operably linked to an expression control sequence are pIFN-γ(-6), pIFN-γ(-7), pIFN-γ(-8), pIFN-γ(-9), pIFN-γ(-10) and pIFN-γ(-11), wherein the coding sequence of the recombinant immune interferon fragments are integrated in the expression vector pDS8/RBSII,SphI (Fig.2). The expression vector pDS8/RSBII,SphI was introduced into E.coli M15 (pDMI,1) a strain of E.coli (E.coli DZ291) transformed with plasmid pDMI,1 (Fig. 4) which plasmid encodes the lac repressor. The resulting E.coli M15 (pDS8/RBSII,SphI; pDMI,1) was deposited on August 6, 1986 at the Deutsche Sammlung von Mikroorganismen (DSM) its accession number being DSM 3809. The methods for the construction of the expression vehicles coding for the recombinant immune interferon fragments IFN-γ(-6), pIFN-γ(-7), IFN-γ(-8), IFN-γ(-9), IFN-γ(-10), and IFN-γ(-11) are outlined in detail in the examples. Based on these examples it is within the skills of an artisan in the field to construct expression vehicles capable of expressing the recombinant immune interferon fragments of the present invention. The replicable expression vehicles capable of expressing recombinant immune interferon fragments are then transformed into a suitable host organism by the specific procedure described in the examples or as described by Maniatis et al. (supra). Depending on the host cell used the recombinant immune interferon fragments may be in glycosylated form or not. Preferred host organisms include Escherichia coli (e.g. strain M15 or strain W3110 [ATCC No. 27325]), Bacillus subtilis and so forth. The most preferred host organism of this invention is E.coli strain M15 mentioned above.

Once the organisms capable of carrying out the expression of the polypeptides of the present invention have been prepared, the process of the invention can be carried out in a variety of ways, depending upon the nature of the construction of the expression vectors and upon the growth characteristics of the host. Typically, the host organism will be grown under conditions which are favorable for the production of large quantities of cells. When a large number of cells has accumulated, suitable inducers or derepressors in the growth medium or a temperature-shift cause the control sequence supplied with such DNA sequence to become active, permitting the transcription and translation of the coding sequence. In the present invention the expression of the gene coding for the recombinant immune interferon fragment is inhibited by the lac repressor. When a large number of cells have accumulated, the interferon gene is derepressed by the addition of isopropyl-$\beta$-D-thiogalactopyranoside (IPTG). The protein produced by the recombinant cell can be released by lysing the cell by conventional means well known in the art. The particular means of lysing will depend upon the host cell utilized. In the present invention lysing the cells with guanidium-hydrochloride is preferred.

The homogeneous recombinant immune interferon fragments of the present invention can be purified by any known method, such as by precipitation with ammonium sulfate, dialysis to remove salts (under normal or vacuum pressure), gel filtration, chromatography, preparative flat-bed iso-electric focusing, gel electrophoresis, high performance liquid chromatography, ion exchange chromatography, gel filtration and reverse phase chromatography, and affinity chromatography and the like, or by using monoclonal or polyclonal antibodies. The homogeneous recombinant immune interferon fragments obtained are essentially free of other proteins as can be judged by SDS-polyacrylamidgel electrophoresis or HPLC analysis.

The recombinant immune interferon fragments may form dimers, trimers or tetramers, that means that the purified recombinant immune interferon fragments may be present as a combination of two, three or four such fragments. The nature of the combining mechanism is unclear.

Antiviral activities of the recombinant immune interferon fragments IFN-$\gamma$(-6), IFN-$\gamma$(-7), IFN-$\gamma$(-8), IFN-$\gamma$(-9), IFN-$\gamma$(-10), and IFN-$\gamma$(-11) were determined in a bioassay based on reduction of the cytopathic effect as described by Rubinstein et al. (J. Virol. 37, 755-758 [1981]). Human amnion cells (FL-cells) and Sindbis virus as a challenge virus were used. Samples were prediluted to about 1000 units/ml and titrated on a microtiter plate in duplicate. A full-length mature recombinant immune interferon [sequence in Fig. 1] preparation (lot No. 85.20 SX-Pool) was used as working standard. It was calibrated to the NIH Reference Human Interferon Gamma Standard, No. Gg 23-901-530. The NIH-Standard has a titer of 3.68 ± 0.34 $\log_{10}$ units/ampoule (n = 39).

The results are summarized in the following Table 1. Each value represents the mean of at least 13 separate determinations. Geometric mean titers were statistically evaluated in a t-test. Differences between the mature recombinant immune interferon and the recombinant immune interferon fragments are highly significant (P= 99%). The difference between the titers of IFN-$\gamma$(-6), and IFN-$\gamma$(-11) are also highly significant. The values of IFN-$\gamma$(-6) and of IFN-$\gamma$(-8) differ significantly (P= 95%). The potencies of IFN-$\gamma$(-8) and IFN-$\gamma$(-11), however, are not significantly different.

**Table 1**

| | Specific antiviral activity |
|---|---|
| Mature recombinant immune interferon (rIFN-$\gamma$) | $9.5 \cdot 10^{6}$ U/mg |
| Recombinant immune interferon fragment IFN-$\gamma$(-6) | $4.1 \cdot 10^{7}$ U/mg |
| Recombinant immune interferon fragment IFN-$\gamma$(-8) | $5.9 \cdot 10^{7}$ U/mg |
| Recombinant immune interferon fragment IFN-$\gamma$(-11) | $7.0 \cdot 10^{7}$ U/mg |

In order to study the increased antiviral activity in more detail, further recombinant immune interferon fragments were constructed either as described above, e.g. IFN-$\gamma$(-4) and IFN-$\gamma$(-5), or by limited proteolysis of full-length mature recombinant immune interferon, e.g. IFN-γ(-14) was prepared using mouse submaxillary gland "Arg C" protease, which cleaves proteins specifically at the carboxylic side of arginine (Schenkein et al., Arch. Biochem. Biophys. 182, 64-70 [1977]), whereas IFN-γ(-18) was prepared using the fibrinolytic enzyme plasmin.

The specific antiviral activity of these polypeptides was determined as described above. Table 2 and Figure 12 show that the recombinant immune interferon fragments IFN-γ(-8), IFN-γ(-9), IFN-γ(-10) and IFN-γ(-11) exhibit a remarkably higher specific antiviral activity than mature recombinant immune interferon or the immune interferon fragments IFN-γ(-14) and IFN-γ(-18) prepared by limited proteolysis.

## Table 2

| | Specific antiviral activity (U/mg) (in brackets 95% confidence limits) |
|---|---|
| rIFN-γ | $1.91 \times 10^7$ ($1.36 \times 10^7$ - $2.67 \times 10^7$) |
| IFN-γ(-4) | $2.88 \times 10^7$ ($2.19 \times 10^7$ - $3.80 \times 10^7$) |
| IFN-γ(-5) | $4.79 \times 10^7$ ($3.54 \times 10^7$ - $6.47 \times 10^7$) |
| IFN-γ(-6) | $3.39 \times 10^7$ ($2.46 \times 10^7$ - $4.66 \times 10^7$) |
| IFN-γ(-8) | $7.08 \times 10^7$ ($5.06 \times 10^7$ - $9.91 \times 10^7$) |
| IFN-γ(-9) | $9.33 \times 10^7$ ($6.60 \times 10^7$ - $1.32 \times 10^8$) |
| IFN-γ(-10) | $8.32 \times 10^7$ ($6.72 \times 10^7$ - $1.03 \times 10^8$) |
| IFN-γ(-11) | $6.31 \times 10^7$ ($4.99 \times 10^7$ - $7.97 \times 10^7$) |

Similarly the immune interferon fragments IFN-γ(-8), IFN-γ(-9), IFN-γ(-10) and IFN-γ(-11) exhibited a remarkably higher antiproliferative activity and increased immunoregulatory activities.

The antiproliferative activity can be determined by measuring the inhibitory action of the different immune interferons on the incorporation of radioactively labeled thymidine into cellular DNA. Three malignant cell lines (U 937, BS 14, LLC 1) and one normal fibroblast line (AG 1523, passage no. 6-10) were used. U 937 is a myelomonocytic leukemia cell line, BS 14 was derived from a patient with malignant melanoma and LLC 1 from a patient with lung cancer. The cells were plated in 96 microwell plates in RPMI 1640 supplemented with 10% fetal calf serum, 1% L-glutamin (200 mM), 1% pencillin (100 u/ml) - streptomycin (100 μg/ml), 1% non-essential amino acids (100 ×) and 1% sodium pyruvate (100 mM) at different densities according to their individual growth curves: 20'000 cells/ml for U 937 and AG 1523, 1'000 cells/ml for BS 14 and LLC 1. The cells were allowed to adhere for 24 hours. Mature recombinant immune interferon (rIFN-γ) and immune interferon fragment IFN-γ(-10) respectively were then added to the wells. Negative control plates were treated with medium alone, whereas for the positive controls, adriamycin was used in a concentration ranging from $10^{-8}$ to $10^{-6}$ M. Two days later $^3$H-thymidine was added to a final concentration of 1 μCi/well. After further 18 hours of incubation the cells were harvested and thymidine incorporation was determined as described by Carr et al. (Cell. Immunol. 5, 21-29 [1972]). It was found that a 14 times lower amount of immune interferon fragment IFN-γ(-10) is needed to obtain the same antiproliferative activity on AG 1523 cells as mature recombinant immune interferon (rIFN-γ). Similarly a 5 times, 3 times and 6 times lower amount of IFN-γ(-10) was found to be needed to obtain the same antiproliferative activity as rIFN-γ on U 937, BS 14 and LLC 1 cells, respectively.

Immune interferon is known to mediate several immunoregulatory functions. It appears to be one of the macrophage-activating factors (MAF). The macrophage activation was measured in two different ways. Normal human macrophages obtained according to Talmadge et al. (Eur. J. Immunol. 16, 1471-1477 [1986]), were activated by the different forms of immune interferon described in the present application. The oxidative burst was measured in terms of enhancement of peroxide release as described by Pick et al. (J. Immunol. Methods 46, 211-226 [1981]), whereas the induction of the bactericidal activity of the macrophages was measured as described by Peck et al. (J. Immunol. Methods 82, 131-140 [1985]). Since immune interferon excerts its immunoregulatory function via binding to membrane receptors, the immunoregulatory activities are dependent on the binding of the immune interferon to its receptor. The competitive binding of radioactively labeled mature recombinant immune interferon and recombinant immune interferon fragments (Kung et al., Meth. in Enzymology 119, 296-301 [1986]) to the interferon receptor was measured as described by Rashidbaigi et al.(J. Biol. Chem. 260, 8514-8519 [1985]). The results of the experiments mentioned above are shown in Figure 13 in summarized form. In addition to the remarkably higher antiviral activity of the immune interferon fragments IFN-γ(-8), IFN-γ(-9), IFN-γ(-10) and IFN-γ(-11), these immune interferon fragments showed also a remarkably increased macrophage activation and receptor binding capacity.

Exposure of the novel recombinant immune interferon fragments to ambient temperature for several days did not generate new peaks in the HPLC analysis as was observed in the case of the full- length mature recombinant immune interferon and therefore in addition to their higher activity these fragments are more stable.

The purified recombinant immune interferon fragments of the present invention or mixtures thereof can be used for the preparation of pharmaceutical compositions. These pharmaceutical compositions contain a significantly reduced amount of said interferon fragments(s), as compared to the amount of mature recombinant human immune interferon needed to obtain essentially the same antiviral activity and a physiological compatible carrier. This reduced amount is based on the significant higher specific antiviral activity (in units/mg) displayed by the recombinant immune interferon fragments (see Table 1). For the preferred recombinant immune interferon fragments of the present invention this reduced amount was calculated using the values for the specific antiviral activity given in Table 1. The reduced amount is about:

23% for recombinant immune interferon fragment IFN-γ(-6)
16% for recombinant immune interferon fragment IFN-γ(-8)
14% for recombinant immune interferon fragment IFN-γ(-11) compared to the amount of mature recombinant immune interferon needed (taken as 100%), to obtain the same specific antiviral activity. Similar results may be obtained using the data of Table 2.

The pharmaceutical compositions can be made up in any conventional form including: a) a solid form for oral administration such as tablets, capsules, pills, powders, granules, and the like; b) a liquid form for oral administration such as solutions, syrups, suspensions, elixirs and the like; c) preparations for parenteral administration such as sterile solutions, suspensions or emulsions; and d) preparations for topical administrations such as solutions, suspensions, ointments, creams, gels, micronized powders, aerosols and the like. The pharmaceutical preparations may be sterilized and/or may contain adjuvants such as preservatives, stabilizers, wetting agents, emulsifiers, salts for varying the osmotic pressure and/or buffers.

Parenteral dosage forms may be infusions or injectable solutions which can be injected intravenously or intramuscularly. These preparations can also contain other medicinally active substances. Additional additives such as preservatives, stabilizers, emulsifying agents, buffers and the like may be added in accordance with accepted practices of pharmaceutical compounding.

In accordance with this invention pharmaceutical compositions containing a recombinant immune interferon fragment can be used for the treatment of viral infections, of neoplastic diseases or of rheumatoid arthritis.

It may be administered in pharmaceutically acceptable oral, injectable or topical composition and modes. Dosage and dose rate may parallel that currently being used in clinical applications of the known interferons, typically about $10^6$ units daily. These pharmaceutical compositions of the invention contain said recombinant immune interferon fragments in association with a compatible pharmaceutically acceptable carrier material. Any conventional carrier material can be utilized. The carrier material can be an organic or inorganic inert carrier material suitable for enteral, percutaneous or parenteral administration. Suitable carriers include water, gelatin, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavoring agents, preservatives, stabilizers, emulsifying agents, buffers and the like may be added in accordance with accepted practices of pharmaceutical compounding.

Having now generally described this invention, the same will become better understood by reference to the specific examples which are included herein for purpose of illustration only and are not intended to be limiting unless otherwise specified.

Example 1

A. Principles

pDS8/RBSII, SphI was chosen for the expression of mature recombinant human immune interferon and the recombinant immune interferon fragments of the present invention. For efficient expression the above mentioned vector contains as an expression control sequence the regulatable promotor/operator element $P_{N25}X_{/0}$ (Stüber et al., EMBO J. 3, 3143-3148 [1984]) and the ribosomal binding site RBSII,SphI. This ribosomal binding site has been derived via DNA-synthesis matching the ribosomal binding site under control of E.coli phage T5 promoter $P_{G25}$ (R. Gentz, Ph.D. Thesis, University of Heidelberg, FRG [1984]). Due to the high efficiency of these expression signals the above mentioned vector can only be stably maintained in E.coli if the promoter/operator element is repressed via binding of the lac repressor to the operator entity of $P_{N25}X_{/0}$. The lac repressor is encoded by the lacI gene. $P_{N25}X_{/0}$ is efficiently repressed only if sufficient amounts of repressor molecules are present. Therefore the $lacI^q$ allel was used, which contains a mutant promoter resulting in an increased expression of the repressor gene. The $lacI^q$ allel is contained in plasmid pDMI,1 (Fig. 4). This plasmid carries in addition to the lacI gene the neo-gene, conferring resistance to kanamycin as a selective marker. Plasmid pDMI,1 is compatible with the expression vector pDS8/RBSII,SphI. E.coli cells to be transformed with expression vectors based on pDS8/RBSII,SphI have to harbour pDMI,1 to ensure stable maintenance of the expression vector. Induction of expression in this system is easily achieved by addition of IPTG to the medium at the desired cell density.

B. Plasmid pDS8/RBSII,SphI

The part of pDS8/RBSII,SphI (Fig. 2) between the sites for restriction endonucleases XbaI and XhoI containing the region required for DNA replication and maintenance of the plasmid in the cell and the entire gene for β-lactamase conferring resistance to ampicillin is derived from plasmid pBR322 (Bolivar et al., Gene 2, 95-113 [(1977]; Sutcliffe supra). The remaining part of the plasmid carries the regulatable promoter/operator element $P_{N25}X_{/0}$ (Stüber et al., supra) followed by the ribosomal binding site RBSII,SphI which is part of an EcoRI/BamHI fragment, by the coding region for dihydrofolate reductase of mouse AT-3000 cell line (Chang et al., Nature 275, 617-624 [1978]; Masters et al., Gene 21, 59-63 [1983]), by the terminator $t_o$ of Escherichia coli phage Lambda (Schwarz et al., Nature 272, 410-414 [1978]), by the promoter-free gene for chloramphenicol acetyltransferase (Marcoli et al., FEBS Letters 110, 11-14 [1980]) and by the terminator T1 of the Escherichia coli rrnB operon (Brosius et al., J.Mol.Biol. 148, 107-127 [1981]-).

C. Plasmid pDMI,1

Plasmid pDMI,1 (Fig. 4) carries the gene for neomycin phosphotransferase from transposon Tn5 (Beck et al., Gene 19, 327-336 [1982]) conferring resistance to kanamycin and the lacI gene (Farabaugh, Nature 274, 765-769 [1978]) with the promoter mutation $I^q$ (Calos, Nature 274, 762-765 [1978]) encoding the lac-repressor. Furthermore, pDMI,1 contains a region of plasmid pACYC184 (Chang et al., J.Bacteriol. 134, 1141-1156 [1978]) carrying the information necessary for its replication and stable maintenance in E.coli.

## Example 2

### Construction of plasmid pGLS encoding IFN-γ

A. Principles

Using chemically synthesized oligonucleotides the IFN-γ gene was adapted to the ribosomal binding site RBSII,SphI (Fig. 6). The fragment encoding IFN-γ was isolated and integrated into pDS8/RBSII,SphI resulting in plasmid pGLS (Fig. 7).

B. Synthesis of the synthetic oligonucleotide fragments adapting the IFNγ-gene to RBSII,SphI.

The synthetic oligonucleotide fragments are shown in Fig. 6. The oligonucleotide fragments themselves were prepared simultaneously on a multisynthesizer as described in European Patent Application No. A1 181 491 published on 21.5.86) using controlled pore glass (CPG) as support material (Kiefer et al., Immunol. Meth. 3, 69-83 [1985]; Sproat et al., Tetrahedr. Lett 24, 5771-5774 [1983]; Adams et al., J. Am. Chem. Soc. 105, 661-663 [1983]). The lyophilised oligonucleotides were dissolved in water for 1 hour at 4°C to give a DNA concentration of 100 nmole/ml.

150 pmoles of each oligonucleotide were kinased with 1 µl of [$^{32}$P]-ATP (2 pmoles, 5000 Ci/mmol), 1 U T4-polynucleotide kinase (Gibco-BRL,Basel) in 10 µl 50mM Tris-HCl pH 8.5, 10 mM $MgCl_2$, for 10 min at 37° C. All reactions were chased by the addition of 1 µl of 5mM ATP. The reactions were stopped by heating the samples for 7 min at 65° C.

C. Construction and isolation of fragment 1 encoding IFN-γ

4 µg of plasmid pRC23/IFI-900 (European Patent Application No. A2 99 084 published on 25.1.84) with a DNA concentration of 400 µg/ml were digested with 10 U of NdeI in BRL-"core buffer" (50 mMTris-HCl, pH 8, 10 mM $MgCl_2$, 50 mM NaCl) supplied by Gibco-BRL in Basle, for 1 h at 37° C in a total volume of 20 µl. After digestion the DNA was once phenol extracted, treated once with ether, precipitated with ethanol and the pellet dried for 2 minutes in a Speed-vac concentrator. The pellet was dissolved in T4-ligase buffer (50 mM Tris/HCl pH 7.8, 10 mM $MgCl_2$, 10 mM DTT, 500 µM ATP. 25 pmoles of the phosphorylated oligonucleotides comprising the SphI-NdeI adapter 1 (Fig. 6) in 1 × ligase buffer were added to a final volume of 25 µl. Ligations were carried out at 22° C for 3 hours using 1 µl DNA-ligase (1 Weiss-unit, Boehringer, Mannheim). The ligation was stopped by incubating the sample for 7 min. at 65° C. The DNA was precipitated with ethanol, dried as described above and then resuspended in 50 µl NcoI digestion buffer (50 mM Tris-HCl, pH 8, 10 mM $MgCl_2$ 50 mM NaCl, 50 mM KCl), 10 U NcoI (BRL-Gibco, Basle) were added and the sample was incubated for 1 hr at 37° C. The restriction enzyme was inactivated by heating the sample for 7 min at 65° C. After a phenol extraction the DNA was precipitated and dried as described above. The pellet was dissolved in 20 µl Klenow-buffer (50 mM Tris-HCl, pH 7.2, 10 mM $MgSO_4$, 100 µM DTT) and dATP, dGTP, dCTP and dTTP were added to a final concentration of 100 µM (total reaction volume: 30 µl). Klenow-enzyme (1 unit, Boehringer Mannheim) was added and the sample was incubated for 1 hr at 22° C. The reaction was stopped by adding 2 µl of 0.25M EDTA. The mixture was extracted once with phenol and the DNA was precipitated as described above and then resuspended in SphI-digestion buffer (50 mM Tris-HCl, pH 7.5, 6mM $MgCl_2$, 50 mM NaCl, 6mM 2-mercaptoethanol). After addition of 10 U SphI (BRL-Gibco, Basle) the sample was incubated for 1 h at 37° C. The digestion was stopped by heating the sample for 7 min at 65° C, followed by one phenol extraction. The DNA was now precipitated and dried as described above.

The DNA-pellet was dissolved in 10 µl gel loading buffer and the DNA fragments electrophoretically separated on a 6% polyacrylamide gel containing 0.5 × Tris-acetate-buffer (40 mM Tris-HCl), 20 mM sodium acetate, 2 mM EDTA, pH 7.8). After staining with ethidiumbromid (0.5 µl/ml) the bands were visualized under 300 nm UV-light. The DNA-band encoding the IFN-γ gene was cut out of the gel with a scalpel. Marker DNA was phage ØX digested with HaeIII (Gibco-BRL, Basle). The gel piece was transferred in a 4×4 mm well in an 0.7% agarose gel containing TBE-buffer (90 mM Tris-borate, 90 mM boric acid, 3 mM EDTA, pH 8.3). The well was sealed with liquid 0.7% agarose in lxTBE in order to give a homogenous electrical field. In front of the sample a piece of NA45 membrane (Schleicher and Schuell, Dassel, BRD) was inserted and the DNA was electrophoresed on the membrane for 5 min at 300 V. The strip with the DNA was then washed with distilled water and transferred into an Eppendorf tube containing 250 µl of 1.5M lithium acetate, 50 mM Tris-HCl pH 8.0 and 10 mM EDTA. The DNA was eluted for 20 min at 65° C with occasional vortexing. The membrane strip was removed from the tube and the sample was once extracted with 200 µl phenol that was saturated with IM Tris pH 8.0. After spinning the samples for 10 min at 12.000 rpm in a microcentrifuge the supernatant containing the DNA was withdrawn. The DNA was precipitated on dry ice for 10 min after adding 20 µl of 5M lithium acetate and 440 µl isopropanol. The DNA was pelleted for 10 min at 12.000 rpm in a microcentrifuge. The pellet was washed with 80% ethanol and dried in vacuo. The pellet was dissolved in 10 µl of TE-buffer (10 mM Tris-HCl pH 7.6, 1 mM EDTA).

D. Preparation of plasmid pDS8/RBSII,SphI

2 pmoles of plasmid pDS8/RBSII,SphI (400 µg/ml were digested with 10 U SphI in "core buffer" at 37° C in a volume of 50 µl for 1 hr. 1 U ScaI was added and the digestion continued for 30 min. After digestion the sample was once phenol extracted, treated with ether, precipitated and dried as described above. The pellet was dissolved in 20 µl buffer (50 mM Tris-HCl, pH 8) containing 1 unit calf intestinal phosphatase (CIP, Boehringer, Mannheim) and incubated for 1 hour at 37° C. After heat-inactivation of the enzyme and removal of the protein (see above) the DNA was precipitated with ethanol. After resuspension of the DNA, the ScaI/SphI fragment of pDS8/RBSII,SphI containing parts of the cat gene, terminator T1, the replication origin, the bla gene, the promoter $P_{N25}x_{/0}$ and the ribosomal binding site RBSII,SphI was isolated via electrophoresis in 1% agarose gels and electrophoretically transfered onto NA45 membranes. Subsequent elution, ethanol precipitation and resuspension in 100 µl TE-buffer was done as described above.

About 1 pmol of the vector fragment was obtained.

E. Assembly of plasmid pGLS

2 µl vector fragment was ligated with 2 µl insert DNA (fragment 1) in ligase buffer (see above) containing 1 µl DNA-ligase (1 Weiss-unit, Boehringer, Mannheim) in a total volume of 20 µl. Ligations were carried out at 22° C for 3 hours. A control ligation with no insert DNA added was done in parallel. The ligations were stopped by heating the samples for 7 min at 65° C. The competent E.coli M15 cells used for the transformation were prepared using the method of Morrison (Methods Enzymol. 68, 326-331 [1979]). The ligation mixtures were added to 200 µl thawed competent E.coli M15 cells harbouring plasmid pDMI,1. The samples were placed on ice for 30 min followed by a 2 min incubation at 42° C. After adding 0.5 ml of LB-medium the samples were incubated for 90 min in a 37° C waterbath. The cells were then centrifuged for 30 sec at 12.000 rpm in a microcentrifuge. The supernatants were withdrawn and the pellets were suspended in 100 µl LB-medium and plated on LB-agar plates containing 100 µg/ml ampicillin plus 25 µg/ml kanamycin. The plates were incubated overnight at 37° C. After transformation of the control ligation no transformants were obtained. The ligation with fragment 1 gave about 200 transformants. Single colonies were picked with a tooth pick, transferred to a tube containing 10 ml LB-medium with 100 µg/ml ampicillin and 25 µg/ml kanamycin and grown for 12 hours at 37° C with vigorous shaking. The cells were centrifuged for 10 min at 8000 rpm. Plasmid DNA was extracted according to the method of Birnboim et al., (Nucleic Acids Res. 7, 1513-1523, [1979]).

The final pellets were dissolved in 200 µl TE-buffer. 4 µl were digested with SphI and XbaI to release the fragment containing the INF-γ gene with the terminator T1. All DNA samples analysed released the fragment of the expected size of about 1 kb. These plasmids were named pGLS.

F. Sequence analysis of the IFN-γ gene, cloned in pGLS

To confirm that the correct IFN-γ sequence is integrated in pGLS, the double-stranded circular plasmid DNA was sequenced using a primer endlabeled with [γ-$^{32}$P]-ATP. The primer contains the nucleotides from position 199-218 of pDS8/RBSII,SphI and ends therefore 6 nucleotides in front of the ATG of the SphI-site. 15 µl of the isolated DNA (0.3 pmol) were precipitated with ethanol and washed once with 80% ethanol. After drying the pellet for 2 min in a Speed vac concentrator the pellet was dissolved in 8 µl 1/4 TE-buffer. After the addition of 2 pmol of the primer, endlabeled with [γ-$^{32}$P]-ATP, the sample was heated for 5 min at 95° C and then placed for 5 min in a 42° C water bath. Now for sequencing of the fragment essentially the dideoxy-chain-termination method described by Sanger et al. (Proc. Natl. Acad. Sci. USA. 74, 5463-5467 [1977]) was used with the modification that since the primer was radioactively labeled, unlabeled deoxyribonucleotide triphosphates (dXTPs) were used in the elongation reaction.

The sequence data proved that the immune interferon gene was properly integrated into plasmid pGLS.

Example 3

Construction of plasmids pIFN-γ(-6), pIFN-γ(-7), pIFN-γ(-8), pIFN-γ(-9), pIFN-γ(-10) and pIFN-γ(-11)

A. Principles

Chemically synthesized oligonucleotides containing translational stop codons at desired positions were ligated to the unique HinfI recognition site of the IFN-γ gene (Fig. 8) resulting finally in fragments F(-6), F(-7), F(-8), F(-9), F(-10) and F(-11). These fragments were ligated into pDS8/RBSII,SphI resulting in the plasmids pIFN-γ(-6), pIFN-γ(-7), pIFN-γ(-8), pIFN-γ(-9), pIFN-γ(-10) and pIFN-γ(-11), (Fig, 9).

B. Preparation of fragments F(-6), F(-7), F(-8), F(-9), F(-10) and F(-11)

3 pmoles of plasmid pGLS were digested with 15 U HinfI (Fig. 8) in "core buffer" at 37° C in a volume of 50 µl for 1 hour. After digestion the restriction enzyme was inactivated for 7 min at 65° C, the sample was phenol extracted, treated with ether, precipitated and dried as described above. The pellet was dissolved in 50 µl ligase-buffer. The oligonucleotides comprising adapters A(-6), A(-7), A(-8), A(-9), A(-10) and A(-11) were synthesized as described in example 2B. 100 pmoles of the phosphorylated adapters were added in parallel to 10 µl of the HinfI digested plasmid pGLS. After the addition of ligase buffer and 1 U of T4-DNA-ligase, ligations were performed in a total volume of 25 µl at 22° C for 12 hours. The reactions were stopped by heating the samples for 7 min at 65° C. After ethanol precipitation the DNA pellets were resuspended in "core buffer" and digested with 15 U EcoRI and 20 U HindIII for 2 hours at 37° C in a volume of 50 µl. After heat inactivation, phenol extraction, treatment with ether and ethanol precipitation, the samples were dissolved in 10 µl gel loading buffer and electrophoresed on a 6% polyacrylamide gel. The DNA bands were visualized under 300 nm UV-light. The bands containing the IFN-γ genes were cut out of the gel and electrophoresed on a NA45 membrane as described above. The marker DNA used was phage ØX-DNA digested with HaeIII. The DNA's were eluted as described and named F(-6), F(-7), F(-8), F(-9), F(-10) and F(-11) respectively.

C. Preparation of plasmid pDS8/RBSII,SphI

As outlined in Fig. 9 2 pmoles of plasmid pDS8/RBSII,SphI were digested with 10 U EcoRI and 10 U HindIII for 1 hour at 37° C in a total volume of 50 µl. After heat inactivation of the enzyme and ethanol precipitation the DNA-pellet was dissolved in 10 µl gel loading buffer. After electrophoresis in a 1% agarose gel the HindIII/EcoRI fragment containing terminator $t_o$, the cat gene, terminator T1, the origin of replication, the bla gene and the promoter $P_{N25}X_{/0}$ was cut out of the gel and eluted as described. The final DNA-pellet was dissolved in 50 µl ligase buffer.

D. Assembly of plasmids pIFN-γ(-6), pIFN-γ(-7), pIFN-γ(-8), pIFN-γ(-9), pIFN-γ(-10) and pIFN-γ(-11)

10 µl of the vector fragment and one half of the amount of each isolated fragments F(-6), F(-7), F(-8), F(-9), F(-10) and F(-11) obtained in Example 3B were ligated in parallel in a volume of 20 µl with 1 unit T4-ligase at 22° C for 3 hours. A control ligation with no insert DNA added was done in parallel. The ligation were stopped by heating the samples for 7 min at 65° C.

Transformations were carried out as described by Morrison (supra) using E.coli M15 harbouring plasmid pDMI.1. The cells were plated on LB-agar plates containing 100 µl/ml ampicillin and 25 µg/ml kanamycin as described above. The plates were incubated overnight at 37° C.

As expected no transformants were obtained in the control ligations. The ligations containing vector DNA plus the fragments gave about 500-1000 colonies. Single colonies were picked with a tooth pick and grown in 10 ml LB-medium as described. Plasmid DNA was extracted according to the method of Birnboim et al., (supra). The final pellets were dissolved in 200 µl TE-buffer. 4 µl of the isolated DNA's were digested with 2 U EcoRI and 2 U HindIII. All clones tested released a fragment of the expected size of about 450 bp.

The plasmids containing fragment F(-6) were named pIFN-γ(-6), whereas the plasmids harbouring F(-7), F(-8), F(-9), F(-10) and F(-11) were named pIFN-γ(-7), pIFN-γ(-8), pIFN-γ(-9), pIFN-γ(-10) and pIFN-γ(-11), respectively. These plasmids are expression vectors for the genes coding for the recombinant immune interferon fragments IFN-γ(-6), IFN-γ(-7), IFN-γ(-8), IFN-γ(-9), IFN-γ(-10) and IFN-γ(-11), respectively (Fig. 10).

## Example 4

### Expression of IFN-γ genes in E.coli

A. Principle

In order to demonstrate that the modified IFN-γ genes are expressed in E.coli in high amounts, they were expressed in E.coli M15 (pDMI,1) transformed with either pIFN-γ(-6), pIFN-γ(-7), pIFN-γ(-8), pIFN-γ(-9), pIFN-γ(-10) or pIFN-γ(-11) and the total cellular protein was analysed by SDS-polyacrylamide gel electrophoresis.

B. Production of IFN-γ-proteins in E.coli

E.coli M15 cells containing plasmid pDMI,1 were transformed with either pIFN-γ(-6), pIFN-γ(-7), pIFN-γ(-8), pIFN-γ(-9), pIFN-γ(-10) or pIFN-γ(-11) and in parallel with pGLS as a control. The transformants were grown in LB-medium containing 100 µg/ml ampicillin and 25 µg/µl kanamycin. At an optical density at 600 nm of about 0.7 the cultures were induced with IPTG (final concentration 1 mM). After additional 6 hours of incubation the cells were harvested by centrifugation.

C. Visualization of proteins produced in E.coli

Cells from a volume of 40 µl of the cultures were resuspended in sample buffer containing 3% SDS, 3% β-mercaptoethanol, 20% glycerol and 125 mM Tris-HCl, pH 6.8. The samples were boiled for 5 minutes, chilled on ice, centrifuged at 12'000 × g for 30 seconds and electrophoresed on a SDS-polyacrylamide gel (12.5% acrylamide, ratio of acrylamide/bisacrylamide of 30/0.8) according to the procedure described by Laemmli (Nature 227, 680-685, [1970]). After staining of the proteins with Coomassie brilliant Blue R-250 the unbound dye was removed from the gel. The gel showed that the recombinant immune interferon fragments IFN-γ(-6), IFN-γ(-7), IFN-γ(-8), IFN-γ(-9), IFN-γ(-10) and IFN-γ(-11) are produced in E.coli in higher amounts than the full-length recombinant mature immune interferon, despite the fact that all proteins are under the control of the same expression control sequence.

Example 5

Purification of the recombinant immune interferon fragments

All recombinant immune interferon fragments were purified to homogeneity by the following method: Recombinant E.coli cells containing an expression vector for a recombinant immune interferon fragment were grown as indicated above. After induction with IPTG and additional 6 hours of incubation the cells were centrifuged (4000 × g, 10 min, 4°C). The supernatant was discarded. Desintegration of the cells (100 g) was performed with 7M guanidine-hydrochloride in 0.01M sodium borate buffer pH 8.0 (300 ml). The resulting suspension was stirred for 1 hour at 4°C. The solubilized recombinant immune interferon fragment was separated from the insoluble particles by centrifugation (10'000 × g, 30 min, 4°C). The supernatant was diluted with a 10-fold volume of 0.15M sodium borate pH 8.0 and the remaining particles were sedimented by centrifugation as before. 360 g silica gel 100 (particle size 0.063-0.200 mm) was added to the crude extract. The suspension was stirred gently to avoid sedimentation. After one hour, the stirring was stopped. Upon formation of a sediment the supernatant was discarded and the sediment transferred to a column (Ø 5 cm, length 17.5 cm). The silica gel in the column was washed with 1M NaCl in 0.01M sodiumborate pH 8.0 (flow-rate 6 ml/min). The recombinant immune interferon fragment was eluted with 0.5M tetramethylammonium chloride and 0.7M ammonium sulfate in 0.01M sodium borate buffer pH 8.0 (flow-rate 6 ml/min). The eluate was immediately pumped on a Phenyl-Sepharose Fast-Flow column (Ø 5 cm, length 21 cm; Pharmacia, Prototyp gel KK33904) which had been equilibrated with 0.5M tetramethylammonium chloride and 0.7M ammonium sulfate in 0.01M sodium borate buffer pH 8.0 (flow-rate 6 ml/min). The recombinant immune interferon fragment was found in the flow through, whereas the contaminating proteases were adsorbed by the matrix. The column was regenerated by washing the matrix with 0.01M sodium borate buffer pH 8.0. In a next step the recombinant immune interferon fragment eluted from the Phenyl-Sepharose column was adsorbed on a Phenyl-Sepharose CL-4B column (Ø 5 cm, length 21 cm, Pharmacia) equilibrated with 0.7M ammonium sulfate in 0.01M sodium borate buffer pH 8.0. The column was washed extensively and the recombinant immune interferon fragment was eluted with a 4 hour linear gradient against 0.01M sodium borate buffer pH 8.0. The eluate was diluted with a 4-fold volume of water and applied on a Fractogel TSK CM-650 (M) column (Ø 2,6 cm, length 18 cm; Merck) equilibrated with pyrogen free 0.05M sodium phosphate buffer pH 6.0 (flow-rate 4 ml/min). The recombinant immune interferon fragment was eluted using a linear NaCl-gradient of 0-0.6M NaCl. For the final purification step the recombinant immune interferon fragment fraction was applied on a Sephadex G-50 superfine column (Ø 5 cm, length 85 cm; Pharmacia), equilibrated with 0.05M sodium phosphate buffer, pH 7.5 (pyrogenfree). The flow-rate was adjusted to 2 ml/min.

The recombinant immune interferon fragments purified in this way were found to be homogeneous upon RP-18 HPLC analysis and SDS-polyacryl amide gel electrophoresis. For C-terminal amino acid analysis the purified proteins were digested with Staphylococcus aureus V8 protease. The C-terminal peptides were isolated on RP-18 HPLC, lyophilized, hydrolyzed in HCl and subjected to amino acid analysis. The expected amino acids were found in the hydrolysate.

After sterile filtration the recombinant immune interferon fragment was found to be stable for several month upon storage at 4°C.

**Claims**

**Claims for the following Contracting States : BE, CH, DE, FR, GB, IT, LI, LU, NL, SE**

**1.** A homogeneous recombinant immune interferon fragment exhibiting a specific antiviral activity higher than mature recombinant immune interferon and having the amino acid sequence

X-Y-Asp-Pro-Tyr-Val-Lys-Glu-Ala-Glu-Asn-Leu-Lys-Lys-Tyr-Phe-
Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-Lys-Asn-Phe-
Lys-Asp-Asp-Gln-Ser-Ile-Gln-Lys-Ser-Val-Glu-Thr-Ile-Lys-Glu-
Asp-Met-Asn-Val-Lys-Phe-Phe-Asn-Ser-Asn-Lys-Lys-Lys-Arg-Asp-
Asp-Phe-Glu-Lys-Leu-Thr-Asn-Tyr-Ser-Val-Thr-Asp-Leu-Asn-Val-
Gln-Arg-Lys-Ala-Ile-His-Glu-Leu-Ile-Gln-Val-Met-Ala-Glu-Leu-
Ser-Pro-Ala-Ala-Lys-Thr-Gly-Lys-Arg-Lys-Arg-Z

wherein either X is a methionine and Y is a glutamine residue, or X is hydrogen and Y is a glutamine or a pyroglutamate residue, and Z is
Ser,
Ser-Gln,
Ser-Gln-Met,
Ser-Gln-Met-Leu,
Ser-Gln-Met-Leu-Phe or
Ser-Gln-Met-Leu-Phe-Arg.

2. A homogeneous recombinant immune interferon fragment according to claim 1 wherein X is a methionine and Y is a glutamine residue.

3. A homogeneous recombinant immune interferon fragment according to claim 1 or 2 wherein Z is Ser-GlnMet-Leu-Phe-Arg.

4. A homogeneous recombinant immune interferon fragment according to claim 1 or 2 wherein Z is Ser-Gln-Met-Leu-Phe.

5. A homogeneous recombinant immune interferon fragment according to claim 1 or 2 wherein Z is Ser-Gln-Met-Leu.

6. A homogeneous recombinant immune interferon fragment according to claim 1 or 2 wherein Z is Ser-Gln-Met.

7. A homogeneous recombinant immune interferon fragment according to claim 1 or 2 wherein Z is Ser-Gln.

8. A homogeneous recombinant immune interferon fragment according to claim 1 or 2 wherein Z is Ser.

9. A homogeneous recombinant immune interferon fragment according to any one of claims 1 to 8 in form of a dimer, a trimer or a tetramer.

10. A replicable microbial expression vehicle comprising a nucleotide sequence, coding upon expression for a recombinant immune interferon fragment as defined in any one of claims 1 to 8, which nucleotide sequence is operably linked to an expression control sequence.

11. A replicable microbial expression vehicle according to claim 10 which is a plasmid which contains inserted into the EcoRI and HindIII cleavage sites of pDS8/RBSII,SphI (DSM 3809) a nucleotide sequence corresponding to the amino acid sequence defined in claim 3, 5 or 8.

12. A replicable microbial expression vehicle according to claim 10 which is a plasmid which contains inserted into the EcoRI and HindIII cleavage sites of pDS8/RBSII,SphI (DSM 3809) a nucleotide sequence corresponding to the amino acid sequence defined in claim 4, 6 or 7.

13. A microorganism transformed with a replicable microbial expression vehicle as claimed in any one of claims 10 to 12, comprising a nucleotide sequence coding upon expression for a recombinant immune interferon fragment according to any one of claims 1 to 8.

14. A recombinant immune interferon fragment according to any one of claims 1 to 9 for the treatment of disease states.

15. A process for the preparation of a recombinant immune interferon fragment according to any one of claims 1 to 9 which process comprises causing a culture of a microorganism, transformed with a replicable expression vehicle comprising a nucleotide sequence coding upon expression for the said interferon fragment, to grow up and express said interferon fragment and recovering it.

16. A process for the preparation of a microorganism according to claim 13 which process comprises transforming a microorganism with a replicable microbial expression vehicle as claimed in any one of claims 10 to 12.

17. A pharmaceutical composition comprising a recombinant immune interferon fragment according to any one of claims 1 to 9 and a physiological compatible carrier.

18. A pharmaceutical composition according to claim 17 for parenteral administration.

19. A pharmaceutical composition according to claim 17 for topical application.

20. A pharmaceutical composition as claimed in claim 17 for intranasal application.

21. The use of a homogeneous recombinant immune interferon fragment as claimed in any one of claims 1 to 9 for the manufacture of a pharmaceutical composition for the treatment of various disease states, such as viral infections, neoplastic diseases and rheumatoid arthritis.

**Claims for the following Contracting States : AT, ES, GR**

1. A process for the preparation of a homogeneous recombinant immune interferon fragment exhibiting a specific antiviral activity higher than mature recombinant immune interferon and having the amino acid sequence

X-Y-Asp-Pro-Tyr-Val-Lys-Glu-Ala-Glu-Asn-Leu-Lys-Lys-Tyr-Phe-
Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-Lys-Asn-Phe-
Lys-Asp-Asp-Gln-Ser-Ile-Gln-Lys-Ser-Val-Glu-Thr-Ile-Lys-Glu-
Asp-Met-Asn-Val-Lys-Phe-Phe-Asn-Ser-Asn-Lys-Lys-Lys-Arg-Asp-
Asp-Phe-Glu-Lys-Leu-Thr-Asn-Tyr-Ser-Val-Thr-Asp-Leu-Asn-Val-
Gln-Arg-Lys-Ala-Ile-His-Glu-Leu-Ile-Gln-Val-Met-Ala-Glu-Leu-
Ser-Pro-Ala-Ala-Lys-Thr-Gly-Lys-Arg-Lys-Arg-Z

wherein either X is a methionine and Y is a glutamine residue, or X is hydrogen and Y is a glutamine or a pyroglutamate residue, and Z is
Ser,
Ser-Gln,
Ser-Gln-Met,
Ser-Gln-Met-Leu,
Ser-Gln-Met-Leu-Phe or
Ser-Gln-Met-Leu-Phe-Arg

which process comprises causing a culture of a microorganism transformed with a replicable expression vehicle, which transformed microorganism is capable of expressing the said interferon fragment, to grow up and express the said interferon fragment and recovering it.

2. A process according to claim 1 characterized in that a culture of a microorganism, transformed with a replicable expression vehicle comprising a nucleotide sequence coding upon expression for a homogeneous recombinant immune interferon fragment as defined in claim 1 wherein X is a methionine and Y is a glutamine residue, is used.

3. A process according to claim 1 characterized in that a culture of a microorganism, transformed with a replicable expression vehicle comprising a nucleotide sequence coding upon expression for a homogeneous recombinant immune interferon fragment as defined in claim 1 or 2 wherein Z is Ser-Gln-Met-Leu-Phe-Arg, is used.

4. A process according to claim 1 characterized in that a culture of a microorganism, transformed with a replicable expression vehicle comprising a nucleotide sequence coding upon expression for a homogeneous recombinant immune interferon fragment as defined in claim 1 or 2 wherein Z is Ser-Gln-Met-Leu-Phe, is used.

5. A process according to claim 1 characterized in that a culture of a microorgansim, transformed with a replicable expression vehicle comprising a nucleotide sequence coding upon expression for a homogeneous recombinant immune interferon fragment as defined in claim 1 or 2 wherein Z is Ser-Gln-Met-Leu, is used.

6. A process according to claim 1 characterized in that a culture of a microorganism, transformed with a replicable expression vehicle comprising a nucleotide sequence coding upon expression for a homogeneous recombinant immune interferon fragment as defined in claim 1 or 2 wherein Z is Ser-Gln-Met, is used.

7. A process according to claim 1 characterized in that a culture of a microorgansim, transformed with a replicable expression vehicle comprising a nucleotide sequence coding upon expression for a homogeneous recombinant immune interferon fragment as defined in claim 1 or 2 wherein Z is Ser-Gln, is used.

8. A process according to claim 1 characterized in that a culture of a microorganism, transformed with a replicable expression vehicle comprising a nucleotide sequence coding upon expression for a homogeneous recombinant immune interferon fragment as defined in claim 1 or 2 wherein Z is Ser, is used.

9. A process according to claim 1 or 2 characterized in that said interferon fragment is recovered in form of a dimer, a trimer or a tetramer.

10. A process for the preparation of a microorganism capable of expressing a recombinant immune interferon fragment as defined in any one of claims 1 to 8 which process comprises transforming a microorganism with a replicable microbial expression vehicle comprising a nucleotide sequence coding upon expression for the said immune interferon fragment, which nucleotide sequence is operably linked to an expression control sequence and growing the transformed microorganism in a culture medium.

11. A method for the preparation of a pharmaceutical composition comprising a homogeneous recombinant immune interferon fragment as defined in any one of claims 1 to 9, which process comprises mixing the said interferon fragment with a compatible pharmaceutically acceptable carrier material.

12. A pharmaceutical composition comprising a homogeneous recombinant immune interferon fragment as defined in any one of claims 1 to 9.

13. The use of a homogeneous recombinant immune interferon fragment as defined in any one of claims 1 to 9 for the manufacture of a pharmaceutical composition for the treatment of various disease states such as viral infections, neoplastic diseases and rheumatoid arthritis.

14. A homogeneous recombinant immune interferon fragment prepared by a process according to any one of claims 1 to 9.

15. A microorganism which is capable of expressing a recombinant human immune interferon fragment as defined in any one of claims 1 to 8 prepared by a process according to claim 10.

16. A replicable microbial expression vehicle comprising a nucleotide sequence, coding upon expression for a recombinant immune interferon fragment as defined in any one of claims 1 to 8, which nucleotide sequence is operably linked to an expression control sequence.

17. A replicable microbial expression vehicle according to claim 16 which is a plasmid which contains inserted into the EcoRI and HindIII cleavage sites of pDS8/RBSII,SphI (DSM 3809) a nucleotide sequence corresponding to the amino acid sequence defined in claim 3, 5 or 8.

18. A replicable microbial expression vehicle according to claim 16 which is a plasmid which contains inserted into the EcoRI and HindIII cleavage sites of pDS8/RBSII,SphI (DSM 3809) a nucleotide sequence corresponding to the amino acid sequence defined in claim 4, 6 or 7.

19. A microorganism transformed with a replicable microbial expression vehicle as claimed in any one of claims 16 to 18, comprising a nucleotide sequence coding upon expression for a recombinant immune interferon fragment as defined in any one of claims 1 to 8.

**Revendications**
**Revendications pour les Etats contractants suivants : BE, CH, DE, FR, GB, IT, LI, LU, NL, SE**

1. Fragment d'interféron immun recombiné homogène présentant une activité antivirale spécifique supérieure à celle de l'interféron immun recombiné mature et ayant la séquence d'amino-acides

X-Y-Asp-Pro-Tyr-Val-Lys-Glu-Ala-Glu-Asn-Leu-Lys-Lys-Tyr-Phe-
Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-Lys-Asn-Phe-
Lys-Asp-Asp-Gln-Ser-Ile-Gln-Lys-Ser-Val-Glu-Thr-Ile-Lys-Glu-
Asp-Met-Asn-Val-Lys-Phe-Phe-Asn-Ser-Asn-Lys-Lys-Lys-Arg-Asp-
Asp-Phe-Glu-Lys-Leu-Thr-Asn-Tyr-Ser-Val-Thr-Asp-Leu-Asn-Val-
Gln-Arg-Lys-Ala-Ile-His-Glu-Leu-Ile-Gln-Val-Met-Ala-Glu-Leu-
Ser-Pro-Ala-Ala-Lys-Thr-Gly-Lys-Arg-Lys-Arg-Z

dans laquelle soit X est méthionine et Y est un reste de glutamine, soit X est un atome d'hydrogène et Y est un reste de glutamine ou de pyroglutamate, et Z est
Ser,
Ser-Gln,
Ser-Gln-Met,
Ser-Gln-Met-Leu,
Ser-Gln-Met-Leu-Phe ou
Ser-Gln-Met-Leu-Phe-Arg.

2. Fragment d'interféron immun recombiné homogène selon la revendication 1, où X est méthionine et Y est un reste de glutamine.

3. Fragment d'interféron immun recombiné homogène selon la revendication 1 ou 2, où Z est Ser-Gln-Met-Leu-Phe-Arg.

4. Fragment d'interféron immun recombiné homogène selon la revendication 1 ou 2, où Z est Ser-Gln-Met-Leu-Phe.

5. Fragment d'interféron immun recombiné homogène selon la revendication 1 ou 2, où Z est Ser-Gln-Met-Leu.

6. Fragment d'interféron immun recombiné homogène selon la revendication 1 ou 2, où Z est Ser-Gln-Met.

7. Fragment d'interféron immun recombiné homogène selon la revendication 1 ou 2, où Z est Ser-Gln.

8. Fragment d'interféron immun recombiné homogène selon la revendication 1 ou 2, où Z est Ser.

9. Fragment d'interféron immun recombiné homogène selon l'une quelconque des revendications 1 à 8 sous forme d'un dimère, d'un trimère ou d'un tétramère.

**10.** Véhicule microbien d'expression réplicable comprenant une séquence nucléotidique codant lors de l'expression pour un fragment d'interféron immun recombiné comme défini dans l'une quelconque des revendications 1 à 8, laquelle séquence nucléotidique est fonctionnellement associée à une séquence de régulation de l'expression.

**11.** Véhicule microbien d'expression réplicable selon la revendication 10 qui est un plasmide qui contient, insérée dans les sites de clivage EcoR I et Hind III de pDS8/RBSII,SphI (DSM 3809), une séquence nucléotidique correspondant à la séquence des aminoacides définie dans les revendications 3, 5 ou 8.

**12.** Véhicule microbien d'expression réplicable selon la revendication 10 qui est un plasmide qui contient, insérée dans les sites de clivage EcoR I et Hind III de pDS8/RBSII,SphI (DSH 3809), une séquence nucléotidique correspondant à la séquence des aminoacides définie dans les revendications 4, 6 ou 7.

**13.** Microorganisme transformé avec un véhicule microbien d'expression réplicable selon l'une quelconque des revendications 10 à 12, comprenant une séquence nucléotidique codant lors de l'expression pour un fragment d'interféron immun recombiné selon l'une quelconque des revendications 1 à 8.

**14.** Fragment d'interféron immun recombiné selon l'une quelconque des revendications 1 à 9 pour le traitement des états pathologiques.

**15.** Procédé pour la préparation d'un fragment d'interféron immun recombiné selon l'une quelconque des revendications 1 à 9, lequel procédé consiste à faire en sorte qu'une culture d'un microorganisme, transformé avec un véhicule microbien d'expression réplicable comprenant une séquence nucléotidique codant lors de l'expression pour ledit fragment d'interféron, se développe et exprime ledit fragment d'interféron, et la récupération de celui-ci.

**16.** Procédé pour la préparation d'un microorganisme selon la revendication 13, lequel procédé comprend la transformation d'un microorganisme avec un véhicule microbien d'expression réplicable selon l'une quelconque des revendications 10 à 12.

**17.** Composition pharmaceutique comprenant un fragment d'interféron immun recombiné selon l'une quelconque des revendications 1 à 9 et un véhicule physiologique compatible.

**18.** Composition pharmaceutique selon la revendication 17 pour l'administration parentérale.

**19.** Composition pharmaceutique selon la revendication 17 pour l'administration locale.

**20.** Composition pharmaceutique selon la revendication 17 pour l'application intranasale.

**21.** Utilisation d'un fragment d'interféron immun recombiné homogène selon l'une quelconque des revendications 1 à 9 pour la préparation d'une composition pharmaceutique pour le traitement de divers états pathologiques, tels que les infections virales, les maladies néoplasiques et la polyarthrite rhumatoïde.

**Revendications pour les Etats contractants suivants : AT, ES, GR**

**1.** Procédé pour la préparation d'un fragment d'interféron immun recombiné homogène présentant une activité antivirale spécifique supérieure à celle de l'interféron immun recombiné mature et ayant la séquence d'amino-acides

X-Y-Asp-Pro-Tyr-Val-Lys-Glu-Ala-Glu-Asn-Leu-Lys-Lys-Tyr-Phe-
Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-Lys-Asn-Phe-
Lys-Asp-Asp-Gln-Ser-Ile-Gln-Lys-Ser-Val-Glu-Thr-Ile-Lys-Glu-
Asp-Met-Asn-Val-Lys-Phe-Phe-Asn-Ser-Asn-Lys-Lys-Lys-Arg-Asp-
Asp-Phe-Glu-Lys-Leu-Thr-Asn-Tyr-Ser-Val-Thr-Asp-Leu-Asn-Val-
Gln-Arg-Lys-Ala-Ile-His-Glu-Leu-Ile-Gln-Val-Met-Ala-Glu-Leu-
Ser-Pro-Ala-Ala-Lys-Thr-Gly-Lys-Arg-Lys-Arg-Z

dans laquelle soit X est méthionine et Y est un reste de glutamine, soit X est un atome d'hydrogène et Y est un reste de glutamine ou de pyroglutamate, et Z est
Ser,
Ser-Gln,
Ser-Gln-Met,
Ser-Gln-Met-Leu,
Ser-Gln-Met-Leu-Phe ou
Ser-Gln-Met-Leu-Phe-Arg

lequel procédé consiste à faire en sorte qu'une culture d'un microorganisme, transformé avec un véhicule microbien d'expression réplicable, lequel microorganisme transformé est capable d'exprimer ledit fragment d'interféron, se développe et exprime ledit fragment d'interféron, et la récupération de celui-ci.

**2.** Procédé selon la revendication 1, caractérisé en ce que l'on utilise une culture d'un microorganisme transformé avec un véhicule d'expression réplicable comprenant une séquence nucléotidique codant lors de l'expression pour un fragment d'interféron immun recombiné homogène comme défini dans la revendication 1, où X est méthionine et Y est un reste de glutamine.

**3.** Procédé selon revendication 1, caractérisé en ce que l'on utilise une culture d'un microorganisme transformé avec un véhicule d'expression réplicable comprenant une séquence nucléotidique codant lors de l'expresssion pour un fragment d'interféron immun recombiné homogène comme défini dans la revendication 1 ou 2, où Z est Ser-Gln-Met-Leu-Phe-Arg.

**4.** Procédé selon la revendication 1, caractérisé en ce que l'on utilise une culture d'un microorganisme transformé avec un véhicule d'expression réplicable comprenant une séquence nucléotidique codant lors de l'expresssion pour un fragment d'interféron immun recombiné homogène comme défini dans la revendication 1 ou 2, où Z est Ser-Gln-Met-Leu-Phe.

**5.** Procédé selon la revendication 1, caractérisé en ce que l'on utilise une culture d'un microorganisme transformé avec un véhicule d'expression réplicable comprenant une séquence nucléotidique codant lors de l'expresssion pour un fragment d'interféron immun recombiné homogène comme défini dans la revendication 1 ou 2, où Z est Ser-Gln-Met-Leu.

**6.** Procédé selon la revendication 1, caractérisé en ce que l'on utilise une culture d'un microorganisme transformé avec un véhicule d'expression réplicable comprenant une séquence nucléotidique codant lors de l'expresssion pour un fragment d'interféron immun recombiné homogène comme défini dans la revendication 1 ou 2, où Z est Ser-Gln-Met.

**7.** Procédé selon la revendication 1, caractérisé en ce que l'on utilise une culture d'un microorganisme transformé avec un véhicule d'expression réplicable comprenant une séquence nucléotidique codant lors de l' expresssion pour un fragment d'interféron immun recombiné homogène comme défini dans la revendication 1 ou 2, où Z est Ser-Gln.

**8.** Procédé selon la revendication 1, caractérisé en ce que l'on utilise une culture d'un microorganisme transformé avec un véhicule d'expression réplicable comprenant une séquence nucléotidique codant lors de l'expresssion pour un fragment d' interféron immun recombiné homogène comme défini dans la revendication 1 ou 2, où Z est Ser.

**9.** Procédé selon la revendication 1 ou 2, caractérisé en ce que ledit fragment d'interféron est récupéré sous forme d'un dimère, d'un trimère ou d'un tétramère.

**10.** Procédé pour la préparation d'un microorganisme capable d'exprimer un fragment d' interféron immun recombiné comme défini dans l'une quelconque des revendications 1 à 8, lequel procédé comprend la transformation d'un microorganisme avec un véhicule microbien d'expression réplicable comprenant une séquence nucléotidique codant lors de l'expression pour ledit fragment d'interféron immun, laquelle séquence nucléotidique est fonctionnellement associée à une séquence régulatrice d'expression, et la multiplication du microorganisme transformé dans un milieu de culture.

11. Procédé pour la préparation d'une composition pharmaceutique comprenant un fragment d' interféron immun recombiné homogène comme défini dans l'une quelconque des revendications 1 à 9, lequel procédé comprend le mélange dudit fragment d'interféron avec un véhicule compatible pharmaceutiquement acceptable.

12. Composition pharmaceutique comprenant un fragment d'interféron immun recombiné homogène comme défini dans l'une quelconque des revendications 1 à 9.

13. Utilisation d'un fragment d'interféron immun recombiné homogène comme défini dans l'une quelconque des revendications 1 à 9 pour la préparation d'une composition pharmaceutique pour le traitement de divers états pathologiques, tels que les infections virales, les maladies néoplasiques et la polyarthrite rhumatoïde.

14. Fragment d'interféron immun recombiné homogène préparé selon le procédé de l'une quelconque des revendications 1 à 9.

15. Microorganisme capable d'exprimer un fragment d'interféron immun humain recombiné comme défini dans l'une quelconque des revendications 1 à 8, préparé selon le procédé de la revendication 10.

16. Véhicule microbien d'expression réplicable comprenant une séquence nucléotidique codant lors de l'expression pour un fragment d'interféron immun recombiné comme défini dans l'une quelconque des revendications 1 à 8, laquelle séquence nucléotidique est fonctionnellement associée à une séquence de régulation de l'expression.

17. Véhicule microbien d'expression réplicable selon la revendication 16 qui est un plasmide qui contient, insérée dans les sites de clivage EcoR I et Hind III de pDS8/RBSII,SphI (DSM 3809), une séquence nucléotidique correspondant à la séquence des aminoacides définie dans les revendications 3, 5 ou 8.

18. Véhicule microbien d'expression réplicable selon la revendication 16 qui est un plasmide qui contient, insérée dans les sites de clivage EcoR I et Hind III de pDS8/RBSII,SphI (DSM 3809), une séquence nucléotidique correspondant à la séquence des aminoacides définie dans les revendications 4, 6 ou 7.

19. Microorganisme transformé avec un véhicule microbien d'expression réplicable selon l'une quelconque des revendications 16 à 18 comprenant une séquence nucléotidique codant lors de l'expression pour un fragment d' interféron immun recombiné, comme défini dans l'une quelconque des revendications 1 à 8.

**Patentansprüche**
**Patentsprüche für folgende Vertragsstaaten : BE, CH, DE, FR, GB, IT, LI, LU, NL, SE**

1. Ein homogenes, rekombinantes Immuninterferonfragment mit einer spezifischen antiviralen Aktivität, die grösser ist als die von reifem, rekombinantem Immuninterferon, und das die Aminosäuresequenz

X-Y-Asp-Pro-Tyr-Val-Lys-Glu-Ala-Glu-Asn-Leu-Lys-Lys-Tyr-Phe-
Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-Lys-Asn-Phe-
Lys-Asp-Asp-Gln-Ser-Ile-Gln-Lys-Ser-Val-Glu-Thr-Ile-Lys-Glu-
Asp-Met-Asn-Val-Lys-Phe-Phe-Asn-Ser-Asn-Lys-Lys-Lys-Arg-Asp-
Asp-Phe-Glu-Lys-Leu-Thr-Asn-Tyr-Ser-Val-Thr-Asp-Leu-Asn-Val-
Gln-Arg-Lys-Ala-Ile-His-Glu-Leu-Ile-Gln-Val-Met-Ala-Glu-Leu-
Ser-Pro-Ala-Ala-Lys-Thr-Gly-Lys-Arg-Lys-Arg-Z

aufweist, worin entweder X für Methionin steht und Y ein Glutaminrest ist, oder X für Wasserstoff steht und Y für ein Glutamin- oder ein Pyroglutaminsäurerest steht, und Z für
Ser,

Ser-Gln,
Ser-Gln-Met,
Ser-Gln-Met-Leu,
Ser-Gln-Met-Leu-Phe oder
Ser-Gln-Met-Leu-Phe-Arg
steht.

2. Ein homogenes, rekombinantes Immuninterferonfragment gemäss Anspruch 1, dadurch gekennzeichnet dass X für Methionin steht und Y ein Glutaminrest ist.

3. Ein homogenes, rekombinantes Immuninterferonfragment gemäss Anspruch 1 oder 2, dadurch gekennzeichnet dass Z für Ser-Gln-Met-Leu-Phe-Arg steht.

4. Ein homogenes, rekombinantes Immuninterferonfragment gemäss Anspruch 1 oder 2, dadurch gekennzeichnet dass Z für Ser-Gln-Met-Leu-Phe steht.

5. Ein homogenes, rekombinantes Immuninterferonfragment gemäss Anspruch 1 oder 2, dadurch gekennzeichnet dass Z für Ser-Gln-Met-Leu steht.

6. Ein homogenes, rekombinantes Immuninterferonfragment gemäss Anspruch 1 oder 2, dadurch gekennzeichnet dass Z für Ser-Gln-Met steht.

7. Ein homogenes, rekombinantes Immuninterferonfragment gemäss Anspruch 1 oder 2, dadurch gekennzeichnet dass Z für Ser-Gln steht.

8. Ein homogenes, rekombinantes Immuninterferonfragment gemäss Anspruch 1 oder 2, dadurch gekennzeichnet dass Z für Ser steht.

9. Ein homogenes, rekombinantes Immuninterferonfragment gemäss einem der Ansprüche 1 bis 8 in Form Dimers, eines Trimers oder eines Tetramers.

10. Ein replizierbarer, mikrobieller Expressionsträger, der eine Nukleotidsequenz enthält, die nach der Expression für ein rekombinantes Immuninterferonfragment gemäss einem der Ansprüche 1 bis 8 kodiert, dadurch gekennzeichnet dass die Nukleotidsequenz operabel an eine Expressionskontrollsequenz gebunden ist.

11. Ein replizierbarer, mikrobieller Expressionsträger gemäss Anspruch 10, der ein Plasmid ist, das eine Nukleotidsequenz enthält, die einer Aminosäuresequenz entspricht, wie sie in den Ansprüchen 3, 5 oder 8 definiert ist und die in die EcoRI and HindIII Schnittstelle von pDS8/RBSII,SphI (DSM 3809) eingebaut ist.

12. Ein replizierbarer, mikrobieller Expressionsträger gemäss Anspruch 10, der ein Plasmid ist, das eine Nukleotidsequenz enthält, die einer Aminosäuresequenz entspricht, wie sie in den Ansprüchen 4, 6 oder 7 definiert ist und die in die EcoRI and HindIII Schnittstelle von pDS8/RBSII,SphI (DSM 3809) eingebaut ist.

13. Ein Mikroorganismus, der mit einem replizierbaren, mikrobiellen Expressionsträger gemäss einem der Ansprüche 10 bis 12 transformiert ist, der eine Nukleotidsequenz enthält, die bei der Expression für ein rekombinantes Immuninterferonfragment gemäss einem der Ansprüche 1 bis 8 kodiert.

14. Ein rekombinantes Immuninterferonfragment gemäss einem der Ansprüche 1 bis 9 zur Behandlung von Krankheiten.

15. Ein Verfahren zur Herstellung eines rekombinanten Immuninterferonfragments gemäss einem der Ansprüche 1 bis 9, dadurch gekennzeichnet dass eine Kultur eines Mikroorganismus, der mit einem replizierbaren Expressionsträger transformiert ist, der eine Nukleotidsequenz enthält, die bei der Expression für ein obengenanntes Immuninterferonfragment kodiert, wachsen gelassen wird und das Immuninterferon exprimiert und gewonnen wird.

16. Ein Verfahren zur Herstellung eines Mikroorganismus gemäss Anspruch 13, dadurch gekennzeichnet dass ein Mikroorganismus mit einem replizierbaren, mikrobiellen Expressionsträger gemäss einem der Ansprüche 10 to 12 transformiert wird.

17. Eine pharmazeutische Zusammensetzung enthaltend ein rekombinantes Immuninterferonfragment gemäss einem der Ansprüche 1 bis 9 und ein physiologisch verträglicher Träger.

18. Eine pharmazeutische Zusammensetzung gemäss Anspruch 17 zur parenteralen Verabreichung.

19. Eine pharmazeutische Zusammensetzung gemäss Anspruch 17 zur topischen Verabreichung.

20. Eine pharmazeutische Zusammensetzung gemäss Anspruch 17 zur intranasalen Verabreichung.

21. Die Verwendung eines rekombinanten Immuninterferonfragments gemäss einem der Ansprüche 1 bis 9, für die Herstellung einer pharmazeutischen Zusammensetung zur Behandlung verschiedener Krankheiten, wie zum Beispiel viralen Infektionen, neoplastischen Erkrankungen oder rheumatischer Arthritis.

**Patentsprüche für folgende Vertragsstaaten : AT, ES, GR**

1. Ein Verfahren zur Herstellung eines homogenen, rekombinanten Immuninterferonfragments mit einer spezifischen antiviralen Aktivität, die grösser ist als die von reifem, rekombinantem Immuninterferon, und das die Aminosäuresequenz

X-Y-Asp-Pro-Tyr-Val-Lys-Glu-Ala-Glu-Asn-Leu-Lys-Lys-Tyr-Phe-
Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-
Gln-Ser-Gln-Ile-Val-Ser-Phe-Tyr-Phe-Lys-Leu-Phe-Lys-Asn-phe-
Lys-Asp-Asp-Gln-Ser-Ile-Gln-Lys-ser-Val-Glu-Thr-Ile-Lys-Glu-
Asp-Met-Asn-Val-Lys-Phe-Phe-Asn-Ser-Asn-Lys-Lys-Lys-Arg-Asp-
Asp-Phe-Glu-Lys-Leu-Thr-Asn-Tyr-Ser-Val-Thr-Asp-Leu-Asn-Val-
Gln-Arg-Lys-Ala-Ile-His-Glu-Leu-Ile-Gln-Val-Met-Ala-Glu-Leu-
Ser-Pro-Ala-Ala-Lys-Thr-Gly-Lys-Arg-Lys-Arg-Z

aufweist, worin entweder X für Methionin steht und Y ein Glutaminrest ist, oder X für Wasserstoff steht und Y für ein Glutamin- oder ein Pyroglutaminsäurerest steht, und Z für
Ser,
Ser-Gln,
Ser-Gln-Met,
Ser-Gln-Met-Leu,
Ser-Gln-Met-Leu-Phe oder
Ser-Gln-Met-Seu-Phe-Arg
steht, dadurch gekennzeichnet dass eine Kultur eines Mikroorganismus, der mit einem replizierbaren Expressionsträger transfomiert ist, und der fähig ist ein obengenanntes Immuninterferonfragment zu exprimieren, wachsen gelassen wird und das Interferonfragment gewonnen wird.

2. Ein Verfahren gemäss Anspruch 1, dadurch gekennzeichnet dass eine Kultur eines Mikroorganismus, der mit einem replizierbaren Expressionsträger transformiert ist, der eine Nukleotidsequenz enthält, die bei der Expression für ein Immuninterferonfragment, wie es in Anspruch 1 definiert ist, kodiert, worin X für Methionin steht and Y ein Glutaminrest ist, verwendet wird.

3. Ein Verfahren gemäss Anspruch 1, dadurch gekennzeichnet dass eine Kultur eines Mikroorganismus, der mit einem replizierbaren Expressionsträger transformiert ist, der eine Nukleotidsequenz enthält, die bei der Expression für ein Immuninterferonfragment kodiert, wie es in Anspruch 1 oder 2 definiert ist und worin Z für Ser-Gln-Met-Leu-Phe-Arg steht, verwendet wird.

4. Ein Verfahren gemäss Anspruch 1, dadurch gekennzeichnet dass eine Kultur eines Mikroorganismus, der mit einem replizierbaren Expressionsträger transformiert ist, der eine Nukleotidsequenz enthält, die bei der Expression für ein Immuninterferonfragment kodiert, wie es in Anspruch 1 oder 2 definiert ist und worin Z für Ser-Gln-Met-Leu-Phe steht, verwendet wird.

5. Ein Verfahren gemäss Anspruch 1, dadurch gekennzeichnet dass eine Kultur eines Mikroorganismus, der mit einem replizierbaren Expressionsträger transformiert ist, der eine Nukleotidsequenz enthält, die bei der Expression für ein Immuninterferonfragment kodiert, wie es in Anspruch 1 oder 2 definiert ist und worin Z für Ser-Gln-Met-Leu steht, verwendet wird.

6. Ein Verfahren gemäss Anspruch 1, dadurch gekennzeichnet dass eine Kultur eines Mikroorganismus, der mit, einem replizierbaren Expressionsträger transformiert ist, der eine Nukleotidsequenz enthält, die bei der Expression für ein Immuninterferonfragment kodiert, wie es in Anspruch 1 oder 2 definiert ist und worin Z für Ser-Gln-Met steht, verwendet wird.

7. Ein Verfahren gemäss Anspruch 1, dadurch gekennzeichnet dass eine Kultur eines Mikroorganismus, der mit einem replizierbaren Expressionsträger transformiert ist, der eine Nukleotidsequenz enthält, die bei der Expression für ein Immuninterferonfragment kodiert, wie es in Anspruch 1 oder 2 definiert ist und worin Z für Ser-Gln steht, verwendet wird.

8. Ein Verfahren gemäss Anspruch 1, dadurch gekennzeichnet dass eine Kultur eines Mikroorganismus, der mit einem replizierbaren Expressionsträger transformiert ist, der eine Nukleotidsequenz enthält, die bei der Expression für ein Immuninterferonfragment kodiert, wie es in Anspruch 1 oder 2 definiert ist und worin Z für Ser steht, verwendet wird.

9. Ein Verfahren gemäss Anspruch 1 oder 2, dadurch gekennzeichnet dass das homogene, rekombinante Immuninterferonfragment in Form eines Dimers, eines Trimers oder eines Tetramers gewonnen wird.

10. Ein Verfahren zur Herstellung eines Mikroorganismus, der fähig ist ein rekombinantes Immuninterferonfragment, wie es in einem der Ansprüche 1 bis 8 definiert ist, zu exprimieren, dadurch gekennzeichnet dass ein Mikroorganismus mit einem replizierbaren, mikrobiellen Expressionsträger transformiert wird, der eine Nukleotidsequenz enthält, die bei der Expression für ein obengenanntes Immuninterferonfragment kodiert, wobei die Nukleotidsequenz operabel an eine Expressionskontrollsequenz gebunden ist und der transformierte Mikroorganismus in eimem Kulturmedium vermehrt wird.

11. Eine Methode zur Herstellung einer pharmazeutischen Zusammensetzung, die ein homogenes, rekombinantes Immuninterferonfragment gemäss einem der Ansprüch 1 bis 9 enthält, dadurch gekennzeichnet, dass ein obengenanntes Interferonfragment mit einem passenden pharmazeutisch verträglichen Träger vermischt wird.

12. Ein pharmazeutische Zusammensetzung enthaltend ein homogenes, rekombinantes Immuninterferonfragment gemäss einem der Ansprüche 1 bis 9.

13. Die Verwendung eines homogenen, rekombinanten Immuninterferonfragments, wie es in einem der Ansprüche 1 bis 9 definiert ist, für die Herstellung einer pharmazeutischen Zusammensetzung zur Behandlung verschiedener Krankheiten, wie zum Beispiel viralen Infektionen, neoplastischen Erkrankungen oder rheumatischer Arthritis.

14. Ein homogenes, rekombinantes Immuninterferonfragment hergestellt nach einem Verfahren gemäss einem der Ansprüche 1 bis 9.

15. Ein Mikroorganismus, der fähig ist ein rekombinantes menschliches Immuninterferonfragment, wie es in einem der Ansprüche 1 bis 8 definiert ist, zu exprimieren, hergestellt nach einem Verfahren gemäss Anspruch 10.

16. Ein replizierbarer, mikrobieller Expressionsträger, der eine Nukleotidsequenz enthält, die bei der Expression für ein rekombinantes Immuninterferonfragment, wie es in einem der Ansprüche 1 bis 8 definiert ist, kodiert, dadurch gekennzeichnet dass die Nukleotidsequenz operabel an eine Expressionskontrollsequenz gebunden ist.

17. Ein replizierbarer, mikrobieller Expressionsträger gemäss Anspruch 16, der ein Plasmid ist, das eine Nukleotidsequenz enthält, die einer Aminosäuresequenz entspricht, wie sie in den Ansprüchen 3, 5 oder 8 definiert ist, wobei die Nukleotidsequenz in die EcoRI and HindIII Schnittstelle von pDS8/RBSII,SphI (DSM 3809) eingebaut ist.

**18.** Ein replizierbarer, mikrobieller Expressionsträger gemäss Anspruch 16, der ein Plasmid ist, das eine Nukleotidsequenz enthält, die einer Aminosäuresequenz entspricht, wie sie in den Ansprüchen 4, 6 oder 7 definiert ist, wobei die Nukleotidsequenz in die EcoRI and HindIII Schnittstelle von pDS8/RBSII,SphI (DSM 3809) eingebaut ist.

**19.** Ein Mikroorganismus, der mit einem replizierbaren, mikrobiellen Expressionsträger gemäss einem der Ansprüche 16 bis 18 transformiert ist, der eine Nukleotidsequenz enthält, die bei der Expression für ein rekombinantes Immuninterferonfragment, wie es in einem der Ansprüche 1 bis 8 definiert ist, kodiert.

# Fig.1

ATG CAG GAC CCA TAT GTA AAA GAA GCA GAA AAC CTT AAG AAA TAT
(Met) Gln Asp Pro Tyr Val Lys Glu Ala Glu Asn Leu Lys Lys Tyr
1

TTT AAT GCA GGT CAT TCA GAT GTA GCG GAT AAT GGA ACT CTT TTC
Phe Asn Ala Gly His Ser Asp Val Ala Asp Asn Gly Thr Leu Phe

TTA GGC ATT TTG AAG AAT TGG AAA GAG GAG AGT GAC AGA AAA ATA
Leu Gly Ile Leu Lys Asn Trp Lys Glu Glu Ser Asp Arg Lys Ile

ATG CAG AGC CAA ATT GTC TCC TTT TAC TTC AAA CTT TTT AAA AAC
Met Gln Ser Gln Ile Val Ser Phe Tyr Phe Lys Leu Phe Lys Asn

TTT AAA GAT GAC CAG AGC ATC CAA AAG AGT GTG GAG ACC ATC AAG
Phe Lys Asp Asp Gln Ser Ile Gln Lys Ser Val Glu Thr Ile Lys

GAA GAC ATG AAT GTC AAG TTT TTC AAT AGC AAC AAA AAG AAA CGA
Glu Asp Met Asn Val Lys Phe Phe Asn Ser Asn Lys Lys Lys Arg

GAT GAC TTC GAA AAG CTG ACT AAT TAT TCG GTA ACT GAC TTG AAT
Asp Asp Phe Glu Lys Leu Thr Asn Tyr Ser Val Thr Asp Leu Asn

GTC CAA CGC AAA GCA ATA CAT GAA CTC ATC CAA GTG ATG GCT GAA
Val Gln Arg Lys Ala Ile His Glu Leu Ile Gln Val Met Ala Glu

HinfI
CTG TCG CCA GCA GCT AAA ACA GGG AAG CGA AAA AGG AGT CAG ATG
Leu Ser Pro Ala Ala Lys Thr Gly Lys Arg Lys Arg Ser Gln Met
130

CTG TTT CGA GGT CGA AGA GCA TCC CAG TAA
Leu Phe Arg Gly Arg Arg Ala Ser Gln

**Fig. 2**

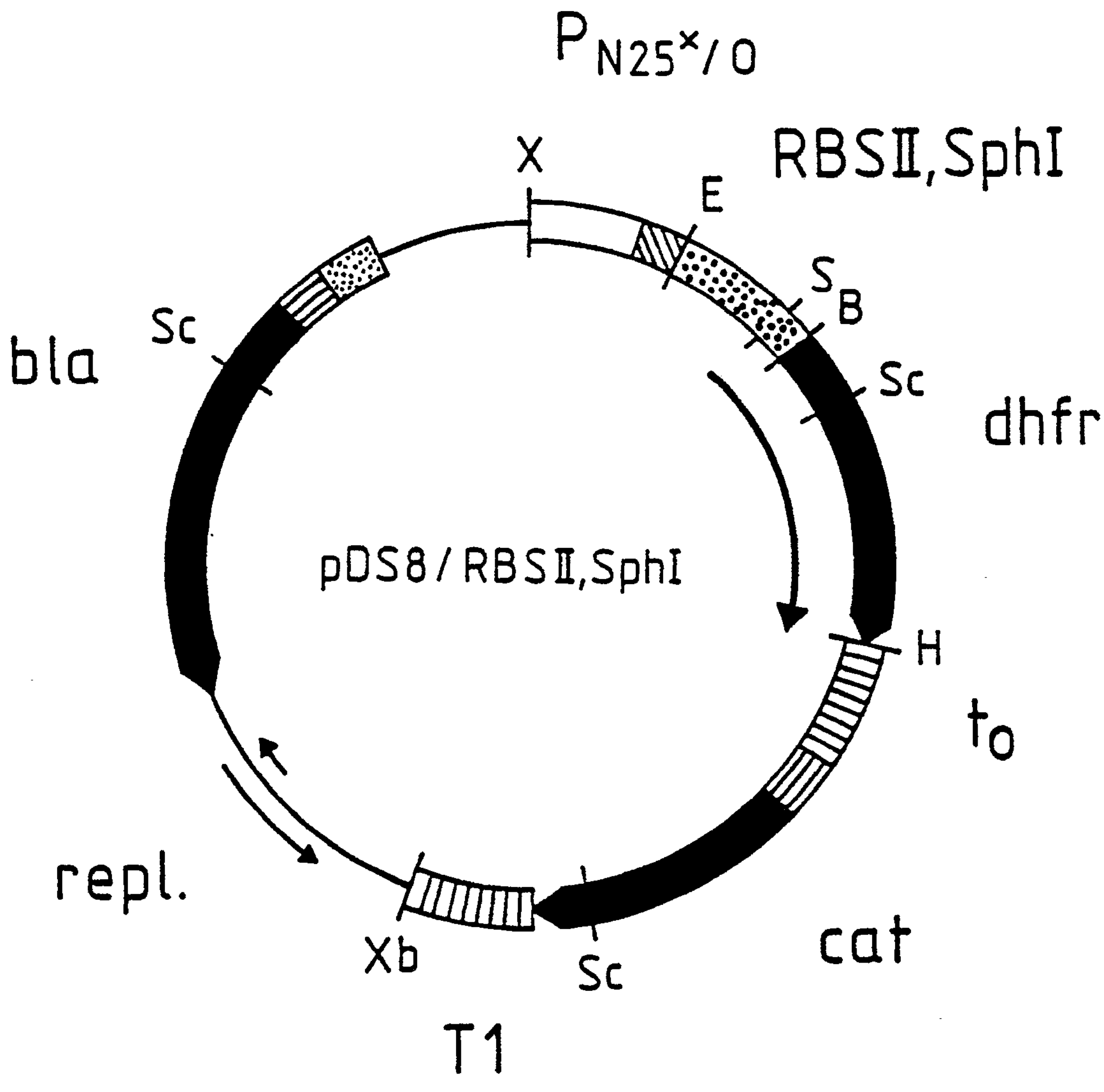
$P_{N25^x/0}$
X
E
RBSII,SphI
S
B
Sc
dhfr
bla
Sc
pDS8/RBSII,SphI
H
$t_o$
repl.
Xb
Sc
cat
T1

# Fig. 3

| | 10 | 20 | 30 | 40 | 50 |
|---|---|---|---|---|---|
| 0 | CCTCGAGGCT | GGCATCCCTA | ACATATCCGA | ATGGTTACTT | AAACAACGGA |
| 50 | GGACTAGCGT | ATCCCTTCGC | ATAGGGTTTG | AGTTAGATAA | AGTATATGCT |
| 100 | GAACTTTCTT | CTTTGCTCAA | AGAATCATAA | AAAATTTATT | TGCTTTCAGG |
| 150 | AAAATTTTTC | TGTATAATAG | ATTCAAATTG | TGAGCGGATA | ACAATTTGAA |
| 200 | TTCATTAAAG | AGGAGAAATT | AAGCATGCGA | GGATCCGGCA | TCATGGTTCG |
| 250 | ACCATTGAAC | TGCATCGTCG | CCGTGTCCCA | AAATATGGGG | ATTGGCAAGA |
| 300 | ACGGAGACCT | ACCCTGGCCT | CCGCTCAGGA | ACGAGTTCAA | GTACTTCCAA |
| 350 | AGAATGACCA | CAACCTCTTC | AGTGGAAGGT | AAACAGAATC | TGGTGATTAT |
| 400 | GGGTAGGAAA | ACCTGGTTCT | CCATTCCTGA | GAAGAATCGA | CCTTTAAAGG |
| 450 | ACAGAATTAA | TATAGTTCTC | AGTAGAGAAC | TCAAAGAACC | ACCACGAGGA |
| 500 | GCTCATTTTC | TTGCCAAAAG | TTTGGATGAT | GCCTTAAGAC | TTATTGAACA |
| 550 | ACCGGAATTG | GCAAGTAAAG | TAGACATGGT | TTGGATAGTC | GGAGGCAGTT |
| 600 | CTGTTTACCA | GGAAGCCATG | AATCAACCAG | GCCACCTTAG | ACTCTTTGTG |
| 650 | ACAAGGATCA | TGCAGGAATT | TGAAAGTGAC | ACGTTTTTCC | CAGAAATTGA |
| 700 | TTTGGGGAAA | TATAAACTTC | TCCCAGAATA | CCCAGGCGTC | CTCTCTGAGG |
| 750 | TCCAGGAGGA | AAAAGGCATC | AAGTATAAGT | TTGAAGTCTA | CGAGAAGAAA |
| 800 | GACTAACAGG | AAGATGCTTT | CAAGTTCTCT | GCTCCCCTCC | TAAAGCTATG |
| 850 | CATTTTTATA | AGACCATGGG | ACTTTTGCTG | GCTTTAGATC | CGGCCAAGCT |
| 900 | TGGACTCCTG | TTGATAGATC | CAGTAATGAC | CTCAGAACTC | CATCTGGATT |
| 950 | TGTTCAGAAC | GCTCGGTTGC | CGCCGGGCGT | TTTTTATTGG | TGAGAATCCA |
| 1000 | AGCTAGCTTG | GCGAGATTTT | CAGGAGCTAA | GGAAGCTAAA | ATGGAGAAAA |
| 1050 | AAATCACTGG | ATATACCACC | GTTGATATAT | CCCAATGGCA | TCGTAAAGAA |
| 1100 | CATTTTGAGG | CATTTCAGTC | AGTTGCTCAA | TGTACCTATA | ACCAGACCGT |
| 1150 | TCAGCTGGAT | ATTACGGCCT | TTTTAAAGAC | CGTAAAGAAA | AATAAGCACA |

# Fig. 3 (cont.)

```
          10         20         30         40         50

1200 AGTTTTATCC GGCCTTTATT CACATTCTTG CCCGCCTGAT GAATGCTCAT
1250 CCGGAATTTC GTATGGCAAT GAAAGACGGT GAGCTGGTGA TATGGGATAG
1300 TGTTCACCCT TGTTACACCG TTTTCCATGA GCAAACTGAA ACGTTTTCAT
1350 CGCTCTGGAG TGAATACCAC GACGATTTCC GGCAGTTTCT ACACATATAT
1400 TCGCAAGATG TGGCGTGTTA CGGTGAAAAC CTGGCCTATT TCCCTAAAGG
1450 GTTTATTGAG AATATGTTTT TCGTCTCAGC CAATCCCTGG GTGAGTTTCA
1500 CCAGTTTTGA TTTAAACGTG GCCAATATGG ACAACTTCTT CGCCCCCGTT
1550 TTCACCATGG GCAAATATTA TACGCAAGGC GACAAGGTGC TGATGCCGCT
1600 GGCGATTCAG GTTCATCATG CCGTCTGTGA TGGCTTCCAT GTCGGCAGAA
1650 TGCTTAATGA ATTACAACAG TACTGCGATG AGTGGCAGGG CGGGGCGTAA
1700 TTTTTTTAAG GCAGTTATTG GTGCCCTTAA ACGCCTGGGG TAATGACTCT
1750 CTAGCTTGAG GCATCAAATA AAACGAAAGG CTCAGTCGAA AGACTGGGCC
1800 TTTCGTTTTA TCTGTTGTTT GTCGGTGAAC GCTCTCCTGA GTAGGACAAA
1850 TCCGCCGCTC TAGAGC——————————————————————————————————
                     |
                    2068

   ———————pBR322———————————————————————————————————A
                                                   |
                                                  4358
```

Fig. 4

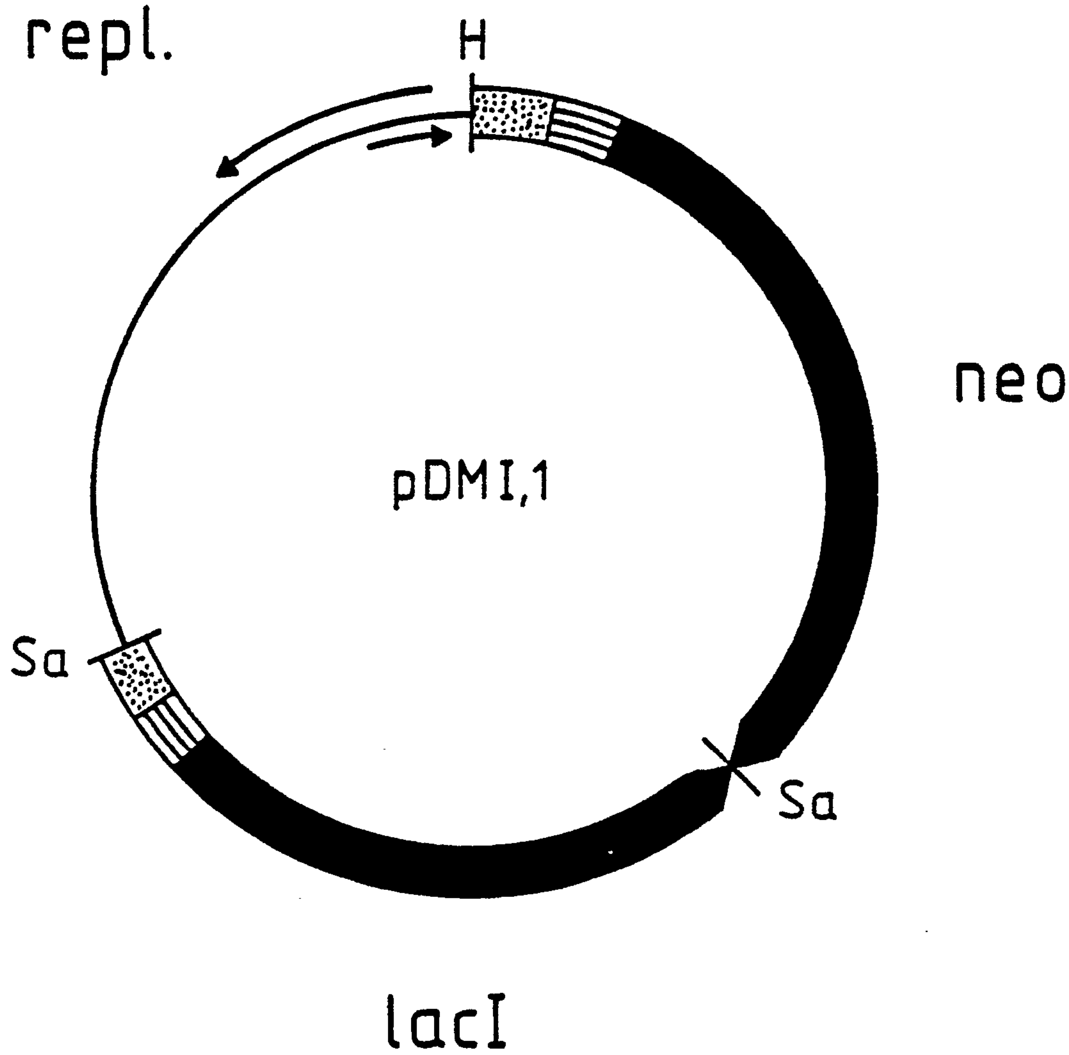

# Fig. 5

10 20 30 40 50

0 AAGCTTCACG CTGCCGCAAG CACTCAGGGC GCAAGGGCTG CTAAAGGAAG
50 CGGAACACGT AGAAAGCCAG TCCGCAGAAA CGGTGCTGAC CCCGGATGAA
100 TGTCAGCTAC TGGGCTATCT GGACAAGGGA AAACGCAAGC GCAAAGAGAA
150 AGCAGGTAGC TTGCAGTGGG CTTACATGGC GATAGCTAGA CTGGGCGGTT
200 TTATGGACAG CAAGCGAACC GGAATTGCCA GCTGGGGCGC CCTCTGGTAA
250 GGTTGGGAAG CCCTGCAAAG TAAACTGGAT GGCTTTCTTG CCGCCAAGGA
300 TCTGATGGCG CAGGGGATCA AGATCTGATC AAGAGACAGG ATGAGGATCG
350 TTTCGCATGA TTGAACAAGA TGGATTGCAC GCAGGTTCTC CGGCCGCTTG
400 GGTGGAGAGG CTATTCGGCT ATGACTGGGC ACAACAGACA ATCGGCTGCT
450 CTGATGCCGC CGTGTTCCGG CTGTCAGCGC AGGGGCGCCC GGTTCTTTTT
500 GTCAAGACCG ACCTGTCCGG TGCCCTGAAT GAACTGCAGG ACGAGGCAGC
550 GCGGCTATCG TGGCTGGCCA CGACGGGCGT TCCTTGCGCA GCTGTGCTCG
600 ACGTTGTCAC TGAAGCGGGA AGGGACTGGC TGCTATTGGG CGAAGTGCCG
650 GGGCAGGATC TCCTGTCATC TCACCTTGCT CCTGCCGAGA AAGTATCCAT
700 CATGGCTGAT GCAATGCGGC GGCTGCATAC GCTTGATCCG GCTACCTGCC
750 CATTCGACCA CCAAGCGAAA CATCGCATCG AGCGAGCACG TACTCGGATG
800 GAAGCCGGTC TTGTCGATCA GGATGATCTG GACGAAGAGC ATCAGGGGCT
850 CGCGCCAGCC GAACTGTTCG CCAGGCTCAA GGCGCGCATG CCCGACGGCG
900 AGGATCTCGT CGTGACCCAT GGCGATGCCT GCTTGCCGAA TATCATGGTG
950 GAAAATGGCC GCTTTTCTGG ATTCATCGAC TGTGGCCGGC TGGGTGTGGC
1000 GGACCGCTAT CAGGACATAG CGTTGGCTAC CCGTGATATT GCTGAAGAGC
1050 TTGGCGGCGA ATGGGCTGAC CGCTTCCTCG TGCTTTACGG TATCGCCGCT
1100 CCCGATTCGC AGCGCATCGC CTTCTATCGC CTTCTTGACG AGTTCTTCTG
1150 AGCGGGACTC TGGGGTTCGA AATGACCGAC CAAGCGACGC CCAACCTGCC
1200 ATCACGAGAT TTCGATTCCA CCGCCGCCTT CTATGAAAGG TTGGGCTTCG
1250 GAATCGTTTT CCGGGACGCC GGCTGGATGA TCCTCCAGCG CGGGGATCTC
1300 ATGCTGGAGT TCTTCGCCCA CCCCGGGCTC GATCCCCTCG CGAGTTGGTT
1350 CAGCTGCTGC CTGAGGCTGG ACGACCTCGC GGAGTTCTAC CGGCAGTGCA
1400 AATCCGTCGG CATCCAGGAA ACCAGCAGCG GCTATCCGCG CATCCATGCC
1450 CCCGAACTGC AGGAGTGGGG AGGCACGATG GCCGCTTTGG TCGACAATTC
1500 GCGCTAACTT ACATTAATTG CGTTGCGCTC ACTGCCCGCT TTCCAGTCGG
1550 GAAACCTGTC GTGCCAGCTG CATTAATGAA TCGGCCAACG CGCGGGGAGA
1600 GGCGGTTTGC GTATTGGGCG CCAGGGTGGT TTTTCTTTTC ACCAGTGAGA
1650 CGGGCAACAG CTGATTGCCC TTCACCGCCT GGCCCTGAGA GAGTTGCAGC
1700 AAGCGGTCCA CGCTGGTTTG CCCCAGCAGG CGAAAATCCT GTTTGATGGT
1750 GGTTAACGGC GGGATATAAC ATGAGCTGTC TTCGGTATCG TCGTATCCCA
1800 CTACCGAGAT ATCCGCACCA ACGCGCAGCC CGGACTCGGT AATGGCGCGC
1850 ATTGCGCCCA GCGCCATCTG ATCGTTGGCA ACCAGCATCG CAGTGGGAAC

# Fig. 5 (cont.)

| | 10 | 20 | 30 | 40 | 50 |
|---|---|---|---|---|---|
| 1900 | GATGCCCTCA | TTCAGCATTT | GCATGGTTTG | TTGAAAACCG | GACATGGCAC |
| 1950 | TCCAGTCGCC | TTCCCGTTCC | GCTATCGGCT | GAATTTGATT | GCGAGTGAGA |
| 2000 | TATTTATGCC | AGCCAGCCAG | ACGCAGACGC | GCCGAGACAG | AACTTAATGG |
| 2050 | GCCCGCTAAC | AGCGCGATTT | GCTGGTGACC | CAATGCGACC | AGATGCTCCA |
| 2100 | CGCCCAGTCG | CGTACCGTCT | TCATGGGAGA | AAATAATACT | GTTGATGGGT |
| 2150 | GTCTGGTCAG | AGACATCAAG | AAATAACGCC | GGAACATTAG | TGCAGGCAGC |
| 2200 | TTCCACAGCA | ATGGCATCCT | GGTCATCCAG | CGGATAGTTA | ATGATCAGCC |
| 2250 | CACTGACGCG | TTGCGCGAGA | AGATTGTGCA | CCGCCGCTTT | ACAGGCTTCG |
| 2300 | ACGCCGCTTC | GTTCTACCAT | CGACACCACC | ACGCTGGCAC | CCAGTTGATC |
| 2350 | GGCGCGAGAT | TTAATCGCCG | CGACAATTTG | CGACGGCGCG | TGCAGGGCCA |
| 2400 | GACTGGAGGT | GGCAACGCCA | ATCAGCAACG | ACTGTTTGCC | CGCCAGTTGT |
| 2450 | TGTGCCACGC | GGTTGGGAAT | GTAATTCAGC | TCCGCCATCG | CCGCTTCCAC |
| 2500 | TTTTTCCCGC | GTTTTCGCAG | AAACGTGGCT | GGCCTGGTTC | ACCACGCGGG |
| 2550 | AAACGGTCTG | ATAAGAGACA | CCGGCATACT | CTGCGACATC | GTATAACGTT |
| 2600 | ACTGGTTTCA | CATTCACCAC | CCTGAATTGA | CTCTCTTCCG | GGCGCTATCA |
| 2650 | TGCCATACCG | CGAAAGGTTT | TGCACCATTC | GATGGTGTCA | ACGTAAATGC |
| 2700 | ATGCCGCTTC | GCCTTCGCGC | GCGAATTGTC | GACCCTGTCC | CTCCTGTTCA |
| 2750 | GCTACTGACG | GGGTGGTGCG | TAACGGCAAA | AGCACCGCCG | GACATCAGCG |
| 2800 | CTAGCGGAGT | GTATACTGGC | TTACTATGTT | GGCACTGATG | AGGGTGTCAG |
| 2850 | TGAAGTGCTT | CATGTGGCAG | GAGAAAAAAG | GCTGCACCGG | TGCGTCAGCA |
| 2900 | GAATATGTGA | TACAGGATAT | ATTCCGCTTC | CTCGCTCACT | GACTCGCTAC |
| 2950 | GCTCGGTCGT | TCGACTGCGG | CGAGCGGAAA | TGGCTTACGA | ACGGGGCGGA |
| 3000 | GATTTCCTGG | AAGATGCCAG | GAAGATACTT | AACAGGGAAG | TGAGAGGGCC |
| 3050 | GCGGCAAAGC | CGTTTTTCCA | TAGGCTCCGC | CCCCCTGACA | AGCATCACGA |
| 3100 | AATCTGACGC | TCAAATCAGT | GGTGGCGAAA | CCCGACAGGA | CTATAAAGAT |
| 3150 | ACCAGGCGTT | TCCCCTGGCG | GCTCCCTCGT | GCGCTCTCCT | GTTCCTGCCT |
| 3200 | TTCGGTTTAC | CGGTGTCATT | CCGCTGTTAT | GGCCGCGTTT | GTCTCATTCC |
| 3250 | ACGCCTGACA | CTCAGTTCCG | GGTAGGCAGT | TCGCTCCAAG | CTGGACTGTA |
| 3300 | TGCACGAACC | CCCCGTTCAG | TCCGACCGCT | GCGCCTTATC | CGGTAACTAT |
| 3350 | CGTCTTGAGT | CCAACCCGGA | AAGACATGCA | AAAGCACCAC | TGGCAGCAGC |
| 3400 | CACTGGTAAT | TGATTTAGAG | GAGTTAGTCT | TGAAGTCATG | CGCCGGTTAA |
| 3450 | GGCTAAACTG | AAAGGACAAG | TTTTGGTGAC | TGCGCTCCTC | CAAGCCAGTT |
| 3500 | ACCTCGGTTC | AAAGAGTTGG | TAGCTCAGAG | AACCTTCGAA | AAACCGCCCT |
| 3550 | GCAAGGCGGT | TTTTTCGTTT | TCAGAGCAAG | AGATTACGCG | CAGACCAAAA |
| 3600 | CGATCTCAAG | AAGATCATCT | TATTAATCAG | ATAAAATATT | TCTAGATTTC |
| 3650 | AGTGCAATTT | ATCTCTTCAA | ATGTAGCACC | TGAAGTCAGC | CCCATACGAT |
| 3700 | ATAAGTTGTT | AATTCTCATG | TTTGACAGCT | TATCATCGAT | |

# Fig. 6

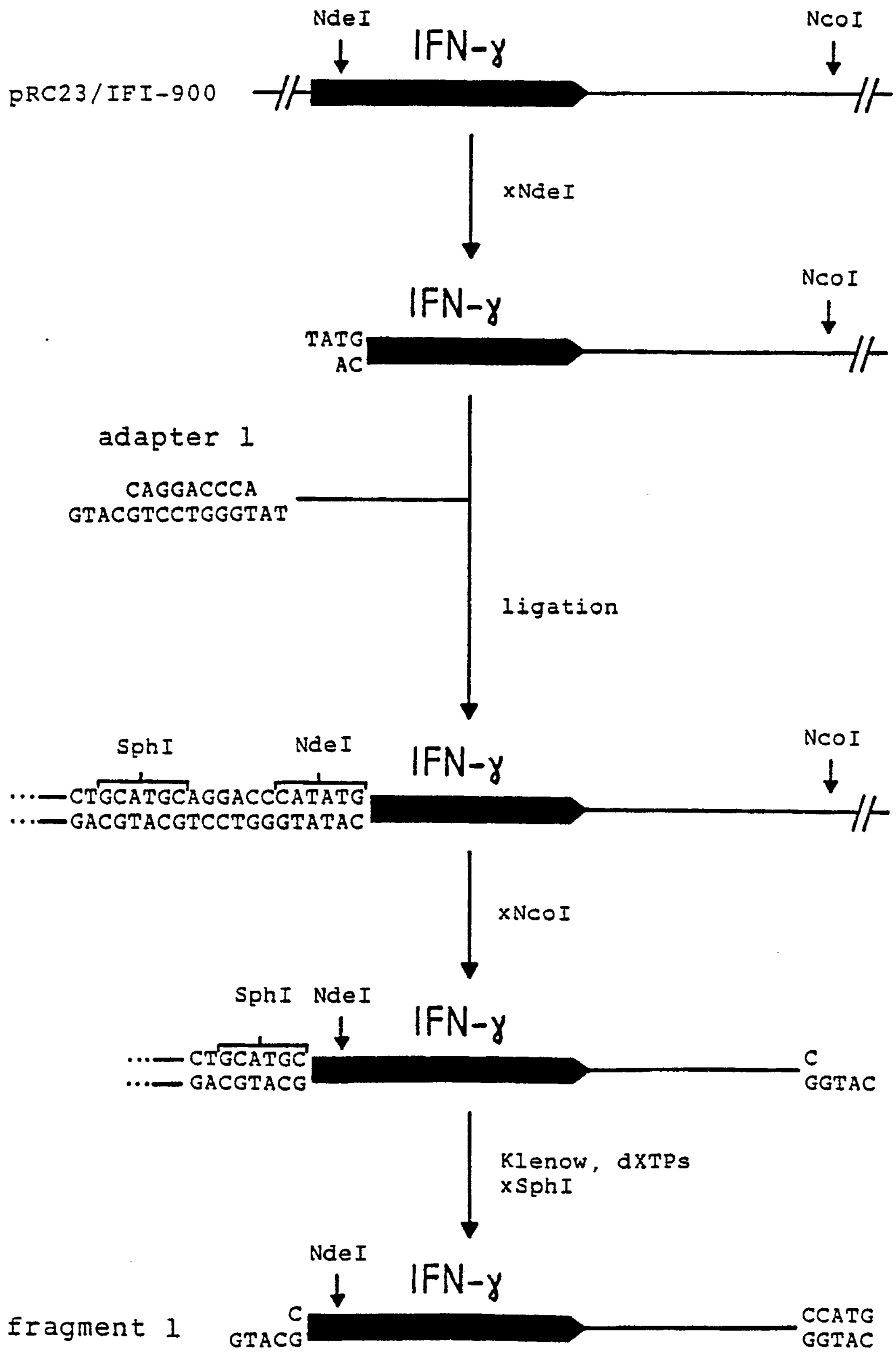
NdeI
IFN-γ
NcoI
pRC23/IFI-900
xNdeI
IFN-γ
NcoI
TATG
AC
adapter 1
CAGGACCCA
GTACGTCCTGGGTAT
ligation
SphI
NdeI
IFN-γ
NcoI
CTGCATGCAGGACCCATATG
GACGTACGTCCTGGGTATAC
xNcoI
SphI
NdeI
IFN-γ
CTGCATGC
GACGTACG
C
GGTAC
Klenow, dXTPs
xSphI
NdeI
IFN-γ
fragment 1
C
GTACG
CCATG
GGTAC

Fig. 7

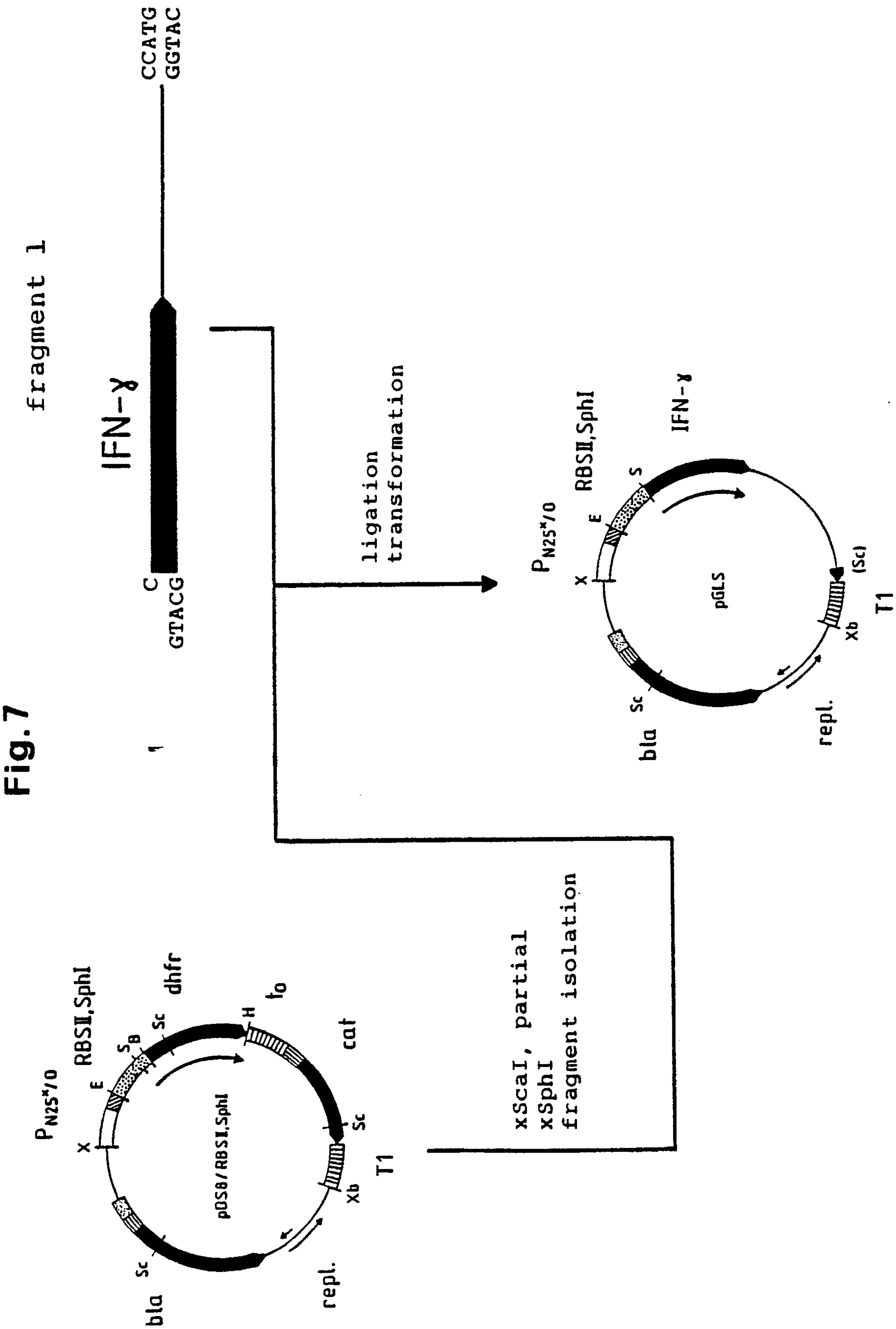
fragment 1
IFN-γ
CCATG
GGTAC
C
GTACG
ligation
transformation
$P_{N25*/0}$
RBSII,SphI
IFN-γ
X
E
S
pGLS
(Sc)
T1
Xb
Sc
bla
repl.
xScaI, partial
xSphI
fragment isolation
RBSII,SphI
dhfr
E
S
B
Sc
H
$t_o$
cat
Sc
T1
Xb
pDS8/RBSII,SphI
repl.
bla

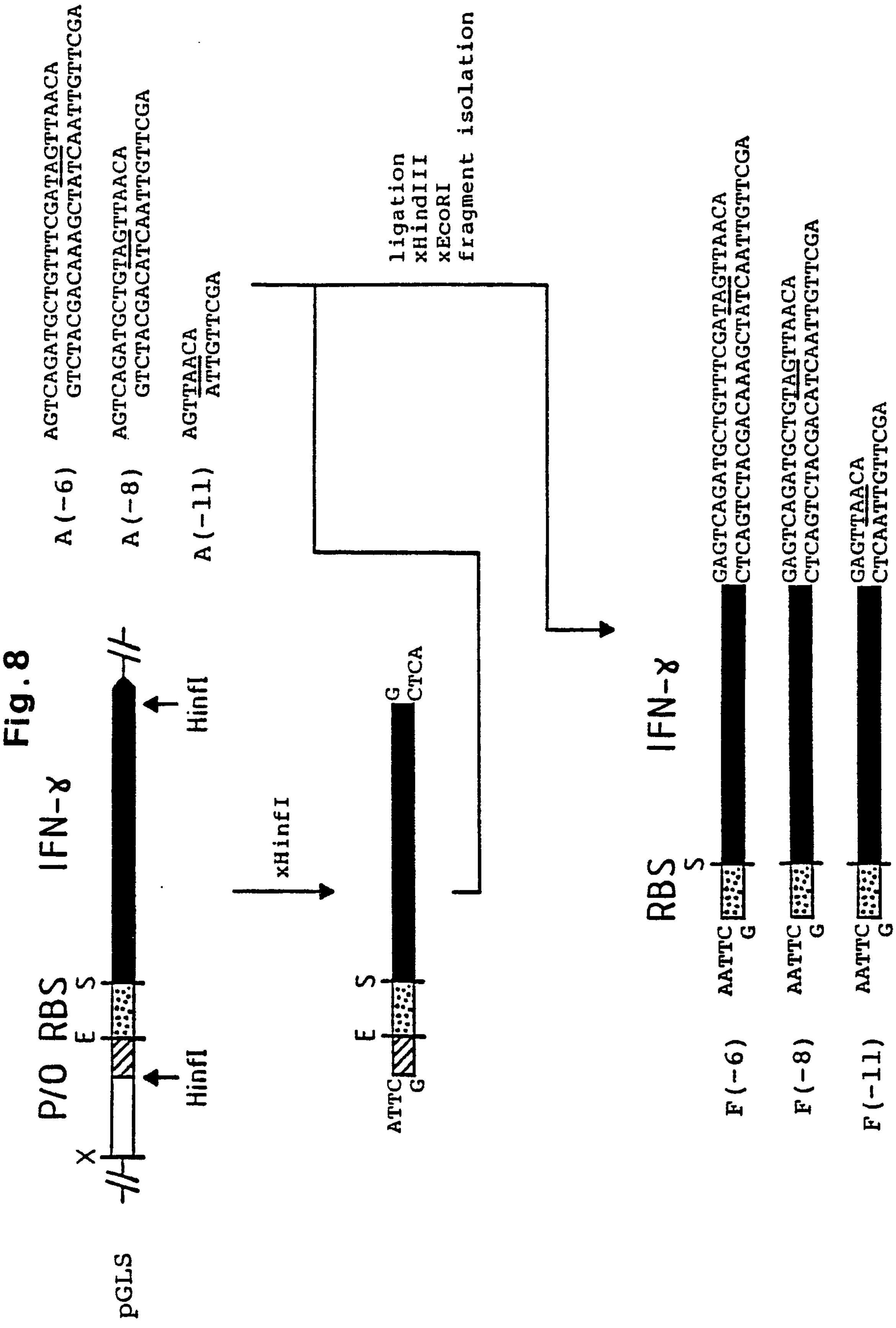
pGLS
P/O RBS
IFN-γ
X
E
S
HinfI
HinfI
xHinfI
ATTC
G
CTCA
A(-6) AGTCAGATGCTGTTCGATAGTTAACA
GTCTACGACAAAGCTATCAATTGTTCGA
A(-8) AGTCAGATGCTGTAGTTAACA
GTCTACGACATCAATTGTTCGA
A(-11) AGTTAACA
ATTGTTCGA
ligation
xHindIII
xEcoRI
fragment isolation
RBS
IFN-γ
F(-6) AATTC
G
GAGTCAGATGCTGTTCGATAGTTAACA
CTCAGTCTACGACAAAGCTATCAATTGTTCGA
F(-8) AATTC
G
GAGTCAGATGCTGTAGTTAACA
CTCAGTCTACGACATCAATTGTTCGA
F(-11) AATTC
G
GAGTTAACA
CTCAATTGTTCGA

Fig. 8

# Fig.9

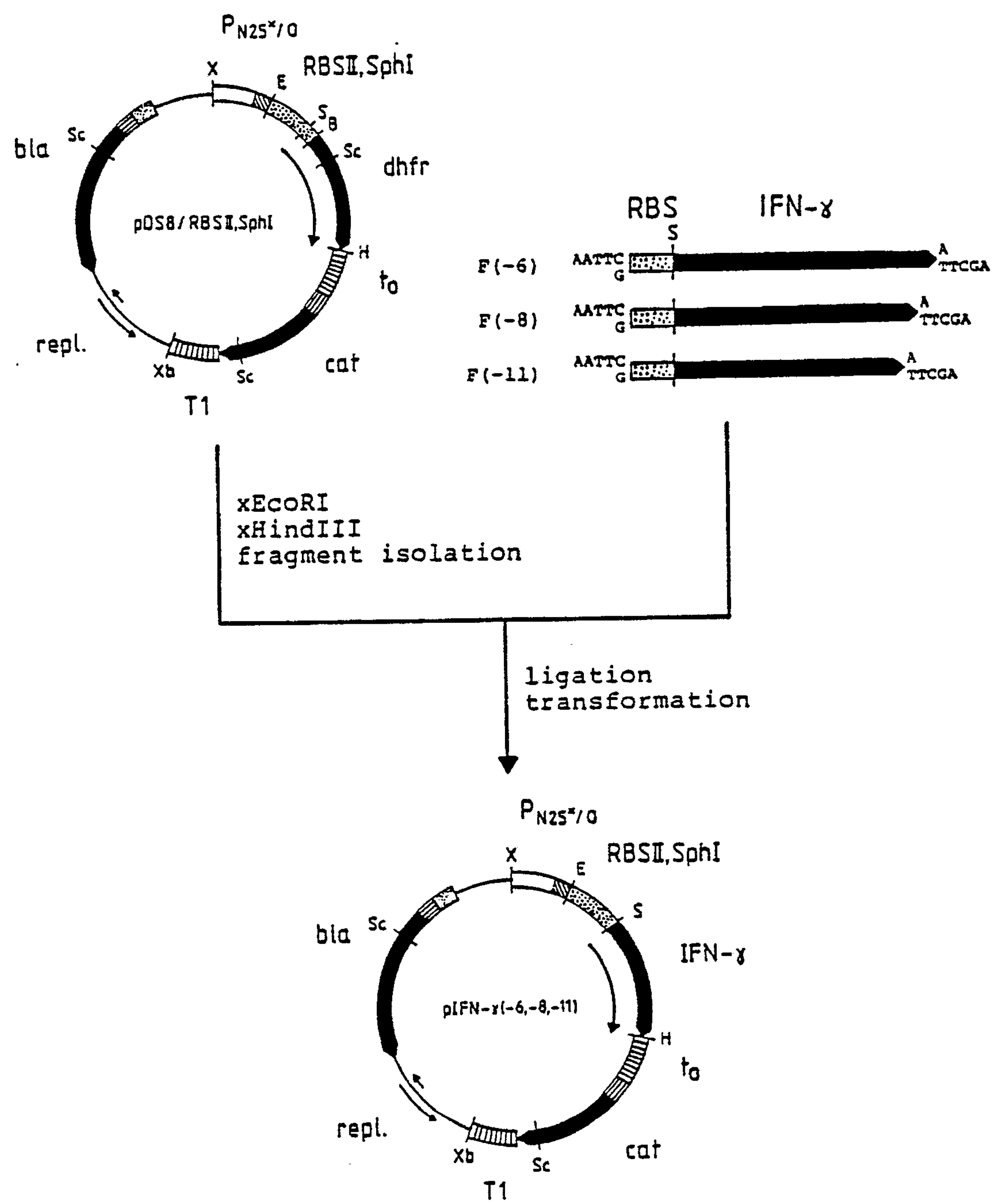
PN25*/O
X
E
RBSII,SphI
SB
Sc
bla
dhfr
pDS8/RBSII,SphI
H
to
repl.
Xb
Sc
cat
T1
RBS
IFN-γ
S
F(-6)
F(-8)
F(-11)
AATTC
G
A
TTCGA
xEcoRI
xHindIII
fragment isolation
ligation
transformation
IFN-γ
pIFN-γ(-6,-8,-11)

Fig. 10

| | |
|---|---|
| rIFN-γ | Met-(130aa)-ArgSerGlnMetLeuPheArgGlyArgArgAlaSerGln |
| IFN-γ(-6) | Met-(130aa)-ArgSerGlnMetLeuPheArg |
| IFN-γ(-7) | Met-(130aa)-ArgSerGlnMetLeuPhe |
| IFN-γ(-8) | Met-(130aa)-ArgSerGlnMetLeu |
| IFN-γ(-9) | Met-(130aa)-ArgSerGlnMet |
| IFN-γ(-10) | Met-(130aa)-ArgSerGln |
| IFN-γ(-11) | Met-(130aa)-ArgSer |

[aa = amino acids]

**Fig.11a**

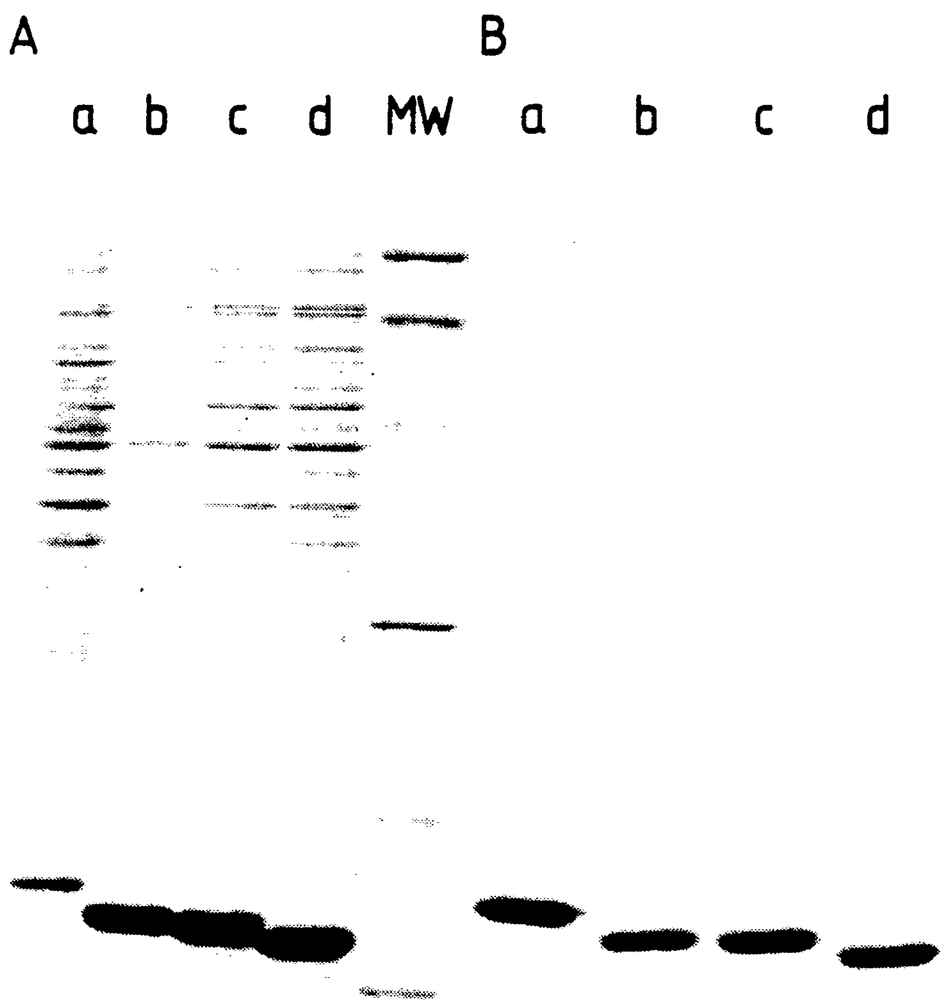

Fig.11b

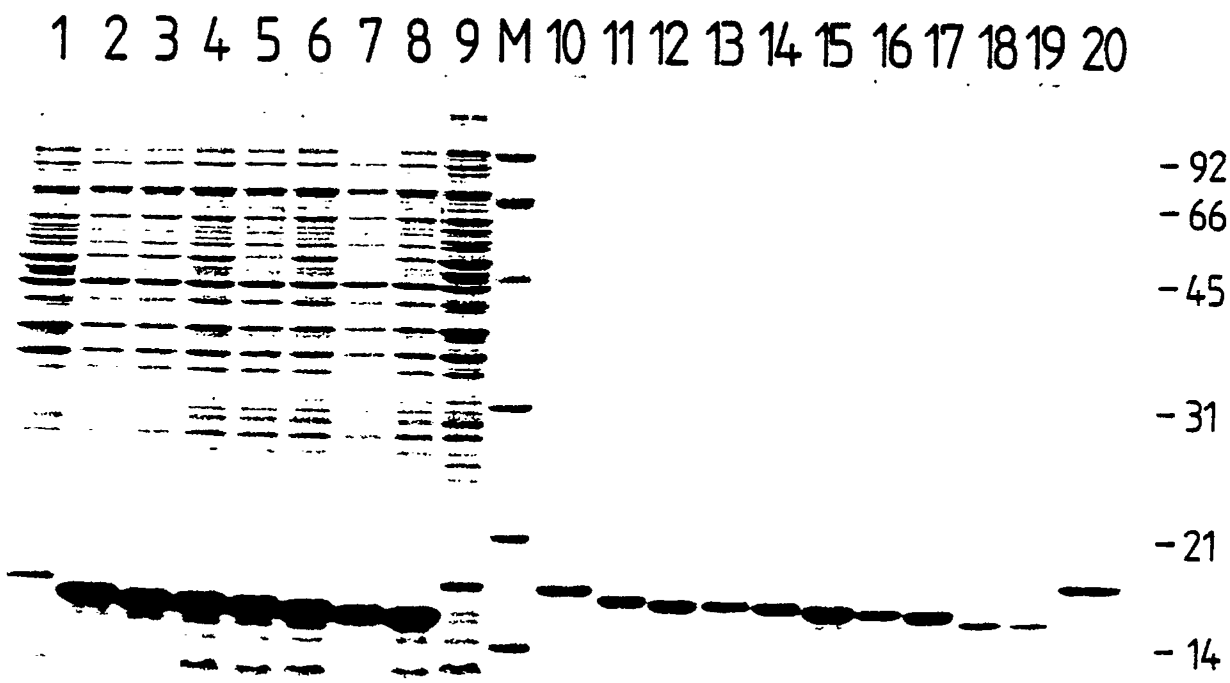

Fig.12

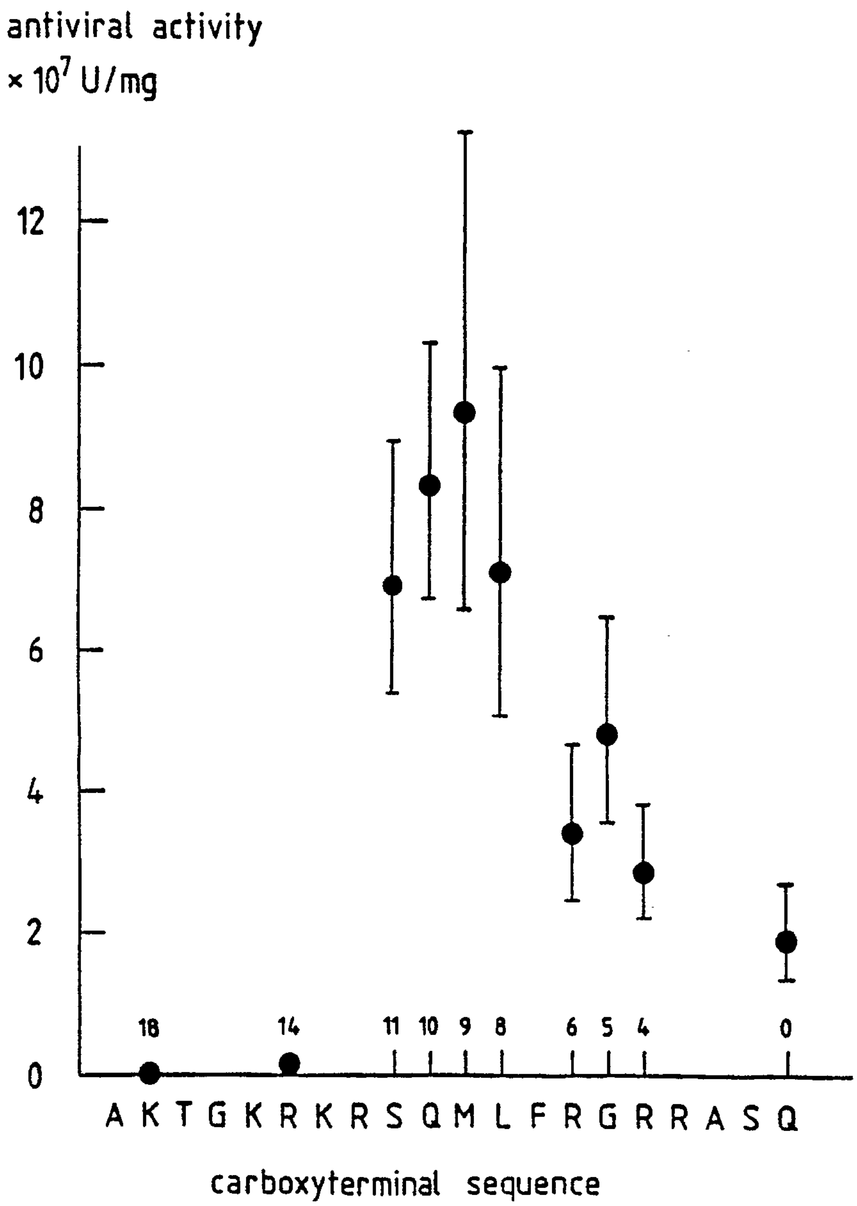
antiviral activity
× 10^7 U/mg
12
10
8
6
4
2
0
18
14
11 10 9 8
6 5 4
0
A K T G K R K R S Q M L F R G R R A S Q
carboxyterminal sequence

Fig. 13

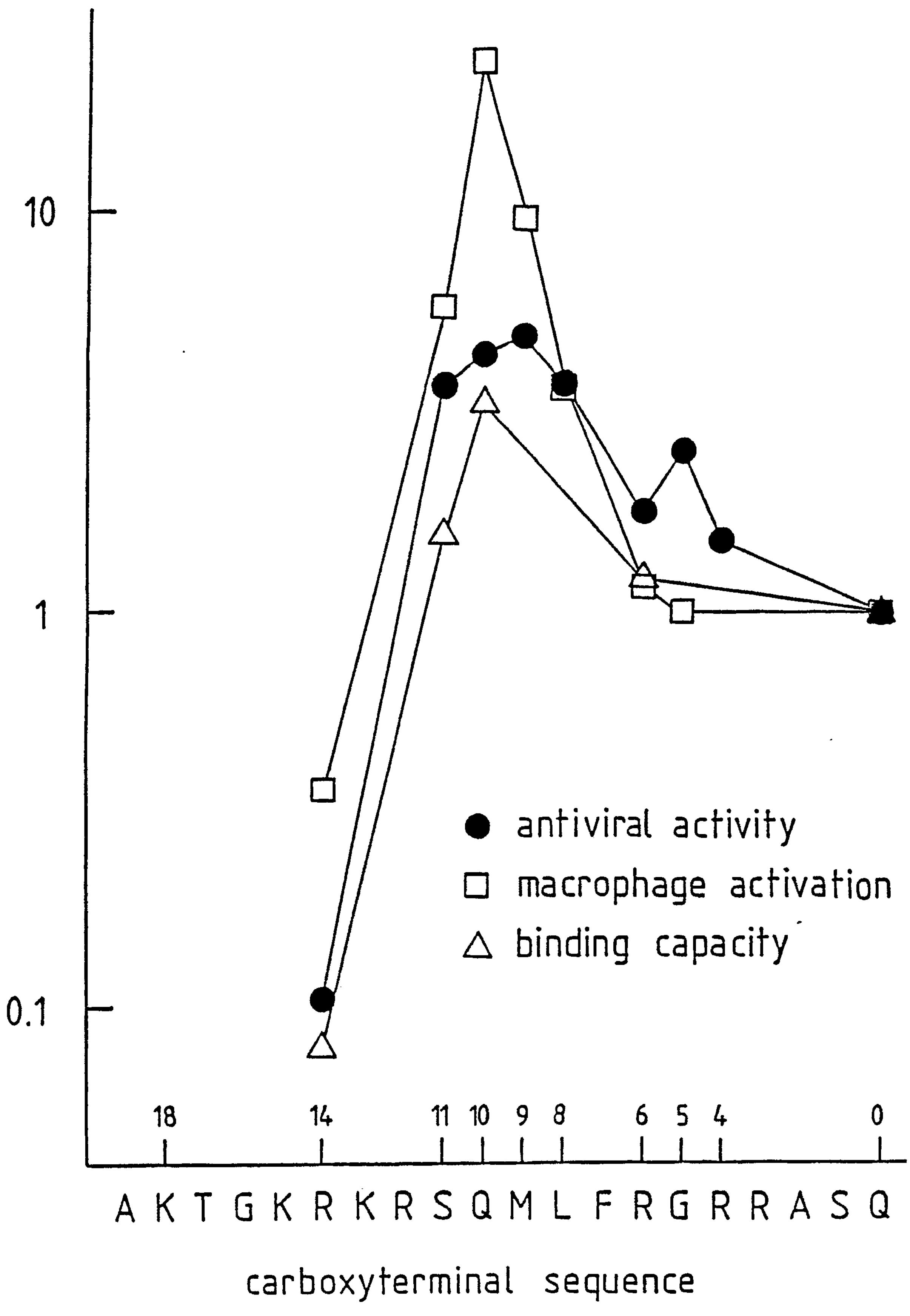
10
1
0.1
antiviral activity
macrophage activation
binding capacity
18
14
11
10
9
8
6
5
4
0
A K T G K R K R S Q M L F R G R R A S Q
carboxyterminal sequence